(19) Europäisches Patentamt
European Patent Office
Office européen des brevets

(11) Publication number: 0 230 110 A1

# (12) EUROPEAN PATENT APPLICATION

(21) Application number: 86309235.9

(22) Date of filing: 26.11.86

(51) Int. Cl.[4]: C 07 D 207/337, C 07 D 231/14, C 07 D 401/06, C 07 D 413/06, A 61 K 31/395

(30) Priority: 30.11.85 GB 8529557
30.11.85 GB 8529558
30.11.85 GB 8529563
30.11.85 GB 8529564
25.04.86 GB 8610218
02.07.86 GB 8616096
02.07.86 GB 8616097
02.07.86 GB 8616100
02.07.86 GB 8616101
02.07.86 GB 8616102
02.07.86 GB 8616103
11.09.86 GB 8621942

(43) Date of publication of application: 29.07.87 Bulletin 87/31

(84) Designated Contracting States: AT BE CH DE ES FR GB GR IT LI LU NL SE

(71) Applicant: FISONS plc, Fison House Princes Street, Ipswich Suffolk IP1 1QH (GB)

(72) Inventor: Baxter, Andrew John Gilby, Flat 4, 6 Western Terrace, The Park Nottingham (GB)
Inventor: Dixon, John, Great Dalby Milton Mowbray Leicestershire, Great Dalby Milton Nowbray Leicestershir (GB)
Inventor: Ince, Francis, 78 Leconfield Road, Loughborough Leicestershire (GB)
Inventor: Springthorpe, Brian, 19 Banbury Drive, Shepshed Leicestershire (GB)
Inventor: Tinker, Alan Charles, 97 Knighttthorpe Road, Loughborough Leicestershire (GB)

(74) Representative: Craig, Christopher Bradberry et al, Fisons plc 12 Derby Road, Loughborough Leicestershire LE11 0BB (GB)

## (54) Pharmacologically active pyrrole and pyrazole derivatives.

(57) There are described compounds of formula I,

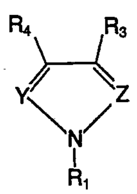

I

wherein

either one of Y and Z (which may be the same or different) represents CH, $CR_2$ or C-$COOR_{21}$, in which $R_2$ represents alkyl C 1 to C6 optionally substituted by halogen, hydroxy, amino, isothioureido or a group $R_5COO$- in which $R_5$ represents alkyl C 1 to C6, $R_2$ being optionally interrupted by O or S, and $R_{21}$ represents alkyl C 1 to C6, and the other one of Y and Z represents $CR_2$ or C-$COOR_{21}$,

or one of Y and Z represents N and the other CH, $CR_2$ or C-$COOR_{21}$,

$R_4$ represents $Ar_1$-B-$(W)_x$, in which

W represents C=O, S, SO, $SO_2$ or C=N–OH,

x represents 0 or 1,

B represents a single bond or $(CH_2)_p$ in which p represents 1 or 2, the group $(CH_2)_p$ being optionally substituted by alkyl C1 to C6, and

$Ar_1$ represents phenyl, naphthyl or benzofurazanyl, $Ar_1$ being optionally substituted by one or more of X, $CX_3$, $OCH_nX_{3-n}$, hydroxy, alkyl C 1 to C6, Oalkyl C 1 to C6, nitro, amino, carboxy, -$SCH_2Ar_2$, -$OCH_2Ar_2$ or -$OCOAr_2$ in which

X represents halogen,

n represents 0, 1, 2 or 3, and

$Ar_2$ represents phenyl or phenyl substituted by hydroxy, halogen, alkyl C 1 to C6 or Oalkyl C 1 to C6, and

$R_3$ and $R_1$ are as defined in the specification.

There are also described processes for the preparation of compounds of formula I and pharmaceutical, eg cardiovascular, compositions containing them.

EP 0 230 110 A1

TITLE MODIFIED
see front page



0230110

HETEROCYCLIC COMPOUNDS

This invention relates to new compounds, new compositions containing them and processes for their preparation.

According to the invention there are provided compounds of formula I,

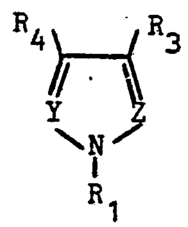

I

wherein

either one of Y and Z, which may be the same or different, represents CH, $CR_2$ or $C-COOR_{21}$, in which $R_2$ represents alkyl C 1 to C6 optionally substituted by halogen, hydroxy, amino, isothioureido or a group $R_5COO-$ in which $R_5$ represents alkyl C 1 to C6, $R_2$ being optionally interrupted by O or S, and $R_{21}$ represents alkyl C 1 to C6, and the other one of Y and Z represents $CR_2$ or $C-COOR_{21}$,

or one of Y and Z represents N and the other CH, $CR_2$ or $C-COOR_{21}$,

$R_4$ represents $Ar_1-B-(W)_x$, in which

W represents C=O, S, SO, $SO_2$ or C=N-OH,

x represents 0 or 1,

B represents a single bond or $(CH_2)_p$ in which p represents 1 or 2, the group $(CH_2)_p$ being optionally substituted by alkyl C 1 to C6, and

$Ar_1$ represents phenyl, naphthyl or benzofurazanyl, $Ar_1$ being optionally substituted by one or more of X, $CX_3$, $OCH_nX_{3-n}$, hydroxy, alkyl C 1 to C6, Oalkyl C 1 to C6, nitro, amino, carboxy, $-SCH_2Ar_2$ or $-OCOAr_2$ in which

X represents halogen,

n represents 0, 1 or 2, and

$Ar_2$ represents phenyl or phenyl substituted by hydroxy, halogen, alkyl C 1 to C6 or Oalkyl C 1 to C6,

and, in addition, when the sum of x and p is 1 or more, or when both Y and Z represent CH, $CR_2$ or $C-COOR_{21}$, then $Ar_1$ may also be singly or multiply substituted by $-OCH_2Ar_2$,

$R_3$ represents $CH_2NR_6R_7$ or $COR_8$, in which $R_6$ and $R_7$, which may be the same or different, represent hydrogen, phenyl, phenyl substituted by halogen or trihalomethyl, alkyl C 1 to C6, or alkyl C 1 to C6 substituted by phenyl, and $R_8$ represents hydrogen or $NR_6R_7$,

and, in addition, when W represents C=O, S=O, $SO_2$ or C=N-OH, or when both Y and Z represent CH, $CR_2$ or $C-COOR_{21}$, then $R_8$ may also represent alkyl C 1 to C6,

OH or $OR_9$ in which $R_9$ represents alkyl C 1 to C6 optionally substituted by an aryl group, and $R_3$ may also represent $NO_2$, CN or halogen, and

$R_1$ represents hydrogen or alkyl C 1 to C6, and pharmaceutically acceptable derivatives thereof.

According to the invention there is also provided a process for the preparation of compounds of formula I, which comprises

a) producing a compound of formula I in which x represents 1 and W represents S or CO by reacting a compound of formula II,

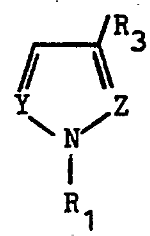

II

in which Y, Z, $R_1$ and $R_3$ are as defined above, with a compound of formula III,

$Ar_1$-B-$W_a$-$L_a$ III

in which $Ar_1$ and B are as defined above, $W_a$ represents S or CO and $L_a$ represents a leaving group, or

b) producing a compound of formula I in which $R_4$ represents $Ar_1$-B-$CH_2$ in which B represents a single

bond or $-CH_2-$ optionally substituted by alkyl C 1 to C6, by reacting a compound of formula II with an aldehyde of formula IV,

$Ar_1-B_b-CHO$ IV

in which $Ar_1$ is as defined above and $B_b$ represents a single bond or $CH_2$ optionally substituted by alkyl C 1 to C6, under reductive alkylation conditions, or

c) producing a compound of formula I in which Z represents $CR_2$, by reacting a compound of formula V,

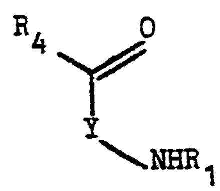

V

in which Y, $R_1$ and $R_4$ are as defined above, with a compound of formula VI,

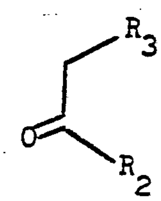

VI

in which $R_2$ and $R_3$ are as defined above, or

d) producing a compound of formula I in which Y represents N by reacting a compound of formula VII,

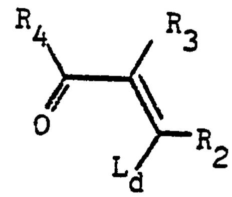

VII

in which $R_2$, $R_3$ and $R_4$ are as defined above and $L_d$ represents a leaving group, with a compound of formula VIII,

$NH_2NHR_1$ VIII

in which $R_1$ is as defined above, and where necessary separating any isomers produced, or

e) producing a compound of formula I in which $R_3$ represents CHO, halogen, $NO_2$, CN or $CH_2NR_6R_7$, by reacting a compound of formula IX,

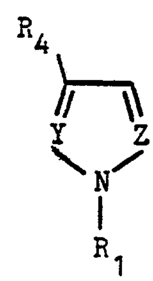

IX

in which Y, Z, $R_1$ and $R_4$ are as defined above, with an appropriate electrophile and, where necessary,

working-up the reaction product, or

f) producing a compound of formula I in which $R_3$ represents $-CONHR_6$ by reacting a compound of formula X,

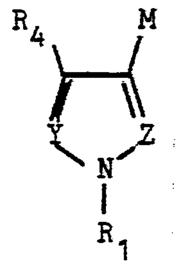

X

in which Y, Z, $R_1$ and $R_4$ are as defined above and M represents a metal, with an isocyanate of formula XI,

$$R_6-N=C=O$$ XI

in which $R_6$ is as defined above, or

g) producing a compound of formula I in which $R_3$ represents $-COR_8$, in which $R_8$ represents $NR_6R_7$ or $OR_9$, by reacting a compound of formula XII,

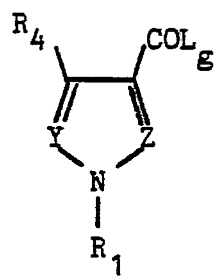

XII

in which Y, Z, $R_1$ and $R_4$ are as defined above and $L_g$ represents a leaving group, with a compound of formula XIII,

$R_{8g}$-H XIII

in which $R_{8g}$ represents $NR_6R_7$ or $OR_9$, or

h) producing a compound of formula I in which $R_3$ represents $NO_2$ and $R_1$ represents hydrogen by rearrangement of a compound of formula XIV,

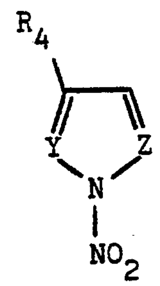

XIV

in which Y, Z and $R_4$ are as defined above, or

i) producing a compound of formula I in which $R_1$ represents hydrogen and one of Y and Z represents nitrogen and the other represents $CR_2$ or C-$COOR_{21}$ by reaction of a compound of formula XV,

$$R_4\text{-}C{\equiv}C\text{-}R_3 \qquad \text{XV}$$

in which $R_3$ and $R_4$ are as defined above with a diazo compound of formula XVI or XVII, as appropriate,

$$R_2\text{-}CH{=}\overset{+}{N}{=}\overset{-}{N} \qquad \text{XVI}$$

$$R_{21}OOC-CH=\overset{+}{N}=\overset{-}{N}$$ XVII

in which $R_2$ and $R_{21}$ are as defined above, and where necessary separating any isomers produced, or

j) producing a compound of formula I in which $R_1$ represents alkyl C 1 to 6 by alkylation of the corresponding compound of formula I in which $R_1$ represents hydrogen, or

k) producing a compound of formula I in which Y represents C-$R_2$ or C-$COOR_{21}$ by reacting a compound of formula XVIII,

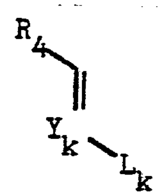

XVIII

in which $R_4$ is as defined above, $Y_k$ represents C-$R_2$ or C-$COOR_{21}$ and $L_k$ represents a leaving group, with a compound of formula XIX,

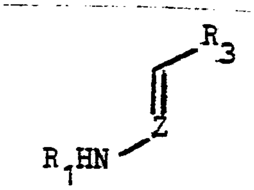

XIX

in which $R_1$, $R_3$ and Z are as defined above, or

l) producing a compound of formula I in which x represents 1 and W represents C=N-OH by treatment of the corresponding compound of formula I in which W represents CO with hydroxylamine, or

m) producing a compound of formula I in which x represents 1 and W represents SO or $SO_2$ by oxidation of the corresponding compound of formula I in which W represents S, or

n) producing a compound of formula I in which one of Y and Z represents CH and the other represents $CR_2$ by reacting a compound of formula XX,

$$R_4-CH=CH-R_3 \qquad XX$$

in which $R_3$ and $R_4$ are as defined above, with a compound of formula XXI,

$$R_2-\underset{\underset{L_n}{|}}{CH}-N\equiv C \qquad XXI$$

in which $R_2$ is as defined above and $L_n$ represents a leaving group, and where necessary separating any isomers produced or

o) producing a compound of formula I in which $Ar_1$ is substituted by one or more $NH_2$ groups by reducing the

corresponding compound of formula I in which $Ar_1$ is substituted by one or more $NO_2$ groups, or

p) producing a compound of formula I in which one of Y and Z represents CH by dealkylation of the corresponding compound of formula I in which one of Y and Z represents $C-R_2$ in which $R_2$ represents alkyl C 1 to C6, or

q) producing a compound of formula I in which $Ar_1$ is substituted by hydroxy by hydrolysis of the corresponding compound of formula I in which $Ar_1$ is substituted by Oalkyl, or

r) producing a compound of formula I in which $Ar_1$ is substituted by $-OCH_2Ar_2$ or $-OCOAr_2$ in which $Ar_2$ is as defined above by reacting the corresponding compound of formula I in which $Ar_1$ is substituted by hydroxy with a compound of formula XXII,

$$Ar_2-X_r-L_r \qquad \text{XXII}$$

in which $Ar_2$ is as defined above, $X_r$ represents CO or $CH_2$ and $L_r$ represents a leaving group or,

s) producing a compound of formula I in which one or both of Y and Z represent $C-R_2$ in which $R_2$ represents alkyl C 1 to C6 substituted by a group $R_5COO-$ by alkanoyloxylation of the corresponding compound of formula I in which $R_2$ represents alkyl C 1 to C6, or

t) producing a compound of formula I in which $R_3$ represents COOH by hydrolysis of the corresponding compound of formula I in which $R_3$ represents $COOR_9$, or

u) producing a compound of formula I in which at least one of Y and Z represents C-$R_2$ in which $R_2$ represents alkyl C 1 to C6 substituted by hydroxy or Oalkyl C 1 to C6 by reacting the corresponding compound of formula I in which $R_2$ represents alkyl C 1 to C6 substituted by $R_5COO$- with a hydroxylic base, and optionally producing a corresponding compound in which $R_2$ is substituted by Oalkyl C 1 to 6 by conducting the reaction in the presence of the corresponding alkanol,

and, where necessary or desired, converting the compound of formula I into a pharmaceutically acceptable derivative thereof, or vice versa.

When W represents CO the reaction of process a) may be carried out in the presence of a Friedel Crafts catalyst, eg $AlCl_3$, in an inert solvent, eg dichloromethane. The reaction is preferably carried out at a temperature of from about -50 to 50°C. Suitable leaving groups that $L_a$ may represent include halide, particularly chloride.

When W represents S, the reaction of process a) may be carried out in a solvent which is inert to the reaction conditions at a temperature from about 0 to 50°C, eg

room temperature. Suitable leaving groups that $L_a$ may represent include halide, particularly chloride.

The reductive alkylation of process b) may be carried out using $H_3PO_2$/HI in acetic acid at room temperature.

The condensation of process c) may be carried out under mildly acidic conditions, eg at a pH of about 4 in water.

The reaction of process d) is preferably carried out in a solvent which is inert to the reaction conditions. Good leaving groups that $L_d$ may represent hydroxy and alkylamino, such as $-NHCH_3$.

It will be appreciated that when the leaving group $L_d$ is hydroxy, the corresponding compound of formula VII may exist in the tautomeric form VIIa:

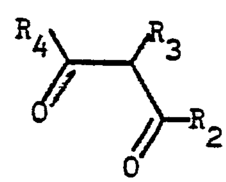

VIIa

In process e), when $R_3$ represents halogen, a suitable electrophile is $Hal^+$ in which Hal represents halogen. The reaction may be carried out using conventional electrophilic halogenation techniques, eg bromination using pyridinium bromide perbromide. The reaction is preferably carried out in a solvent which is

inert to the reaction conditions at a temperature of from about -50 to 50°C.

In process e), when $R_3$ represents CHO, the reaction may be carried out under Vilsmeier-Haack conditions, ie using phosphorous oxychloride and a disubstituted formamide. The reaction is preferably performed in a solvent which is inert to the reaction conditions at a temperature of from about -50 to 50°C.

When $R_3$ represents $NO_2$, an appropriate electrophile is $NO_2^+$. Nitrating agents suitable for use in process e) include nitronium salts such as nitronium tetrafluoroborate. The reaction is preferably carried out in an inert solvent at a temperature of from about -100 to 0°C.

In process e), when $R_3$ represents CN the reaction may be performed using chlorosulphonyl isocyanate followed by treatment with dimethylformamide. The reaction is preferably carried out at a temperature of from about -100 to 0°C in an inert solvent.

When $R_3$ represents $-CH_2NR_6R_7$, the reaction of process e) may be carried out under Mannich conditions, eg using paraformaldehyde and an amine of formula XIII in which $R_{8g}$ represents $NR_6R_7$. The reaction is preferably carried out in a polar solvent at a temperature of from about 0 to 100°C.

Metals that M may represent in process f) include lithium. When M represents lithium, the compound of formual X may be prepared by treatment with n-butyl lithium of the corresponding compound of formula X in which M represents halogen, eg bromine. Compounds of formula X in which M represents halogen may be prepared by the method of process e).

Good leaving groups that $L_g$ may represent in process g) include, for example, halide, eg chloride or bromide, 1-imidazolyl, trifluoromethane sulphonate, alkyl carbonate, eg ethyl or benzyl carbonate, alkanoyloxy, eg trifluoroacetoxy or alkoxy, eg methoxy. When $R_{8g}$ represents $NR_6R_7$, the reaction may be carried out in a solvent which is inert to the reaction conditions, for example a chlorinated hydrocarbon, eg chloroform. The reaction may be carried out at a temperature of from about 0 to 100$^oC$. When $R_8$ represents $OR_9$ the reaction may be carried out in an excess of the compound of formula XIII, ie the compound of formula XIII may act as the solvent. In this case, the reaction may be carried out at a temperature of from about 0 to 100$^oC$.

The rearrangement of process h) is preferably brought about thermally. The reaction is preferably carried out in a solvent which is inert to the reaction conditions at a temperature of from about 50 to 150$^oC$.

The reaction of process i) is preferably carried out in a solvent which is inert to the reaction conditions, eg ether. The reaction is preferably carried out at a temperature of from about -50°C to 50°C.

The reaction of process j) is preferably performed by treatment with an alkali metal hydride, such as sodium hydride, followed by treatment with an alkyl halide, such as an alkyl chloride, bromide or iodide. The reaction is preferably carried out in a solvent which is inert to the reaction conditions, at a temperature of from about 0 to 100°C.

The reaction of process k) is preferably carried out in a polar solvent at a temperature of from about 0 to 100°C. Good leaving groups that $L_k$ may represent include nitro, hydroxy and halide.

The reaction of process l) is preferably performed in the presence of a weak base in a polar solvent. The reaction is preferably carried out at a temperature of from about 50 to 100°C.

The reaction of process m) is preferably performed in an inert solvent at a temperature of from about 0 to 100°C, more preferably 0 to 50°C. Oxidising agents which may be used include 3-chlorobenzeneperoxoic acid. Compounds of formula I in which W represents SO and $SO_2$ may be produced by using about 1.1 and about 3 equivalents

of oxidising agent respectively.

The reaction of process n) is preferably carried out in an inert solvent at a temperature of from about -50 to 50°C. Leaving groups that $L_n$ may represent include (4-methyl)phenylsulphonyl. Compounds of formula XX may be prepared by a Wittig reaction.

The reaction of process o) is preferably carried out by hydrogenation in a polar solvent using a suitable catalyst, eg platinum oxide.

The reaction of process p) may be carried out using conditions similar to those used for the Friedel-Crafts acylation of process a), eg using $AlCl_3$ in an inert solvent, eg dichloromethane, at a temperature of from about -50 to 50°C. In general, however, the required reaction time is much longer, eg 3 days.

The reaction of process q) may be carried out using boron trichloride. The reaction is preferably performed in an inert solvent such as dichloromethane at a temperature of from about -100 to 0°C.

When $X_r$ represents CO, the reaction of process r) may be carried out under similar conditions to the Friedel-Crafts acylation of process a), ie using $AlCl_3$ in an inert solvent, eg dichloromethane. The reaction is preferably carried out at a temperature of from about -100 to 0°C.

When $X_r$ represents $CH_2$, the reaction of process r) may be carried out using sodium hydride. The reaction is preferably carried out in an inert solvent, eg dimethylformamide, at a temperature of from about 0 to 100°C, more preferably 0 to 50°C, eg room temperature.

The alkanoloxylation of process s) may be carried out using a metallic alkanoate. For example, when $R_5$ represents methyl the reaction may be carried out using lead tetraacetate. The reaction is preferably carried out at a temperature of from about 0 to 100°C.

The reaction of process t) may be carried out using trimethylsilyl chloride and sodium iodide. The reaction is preferably carried out at a temperature of from about 50 to 150°C.

Suitable hydroxylic bases for use in process u) include lithium hydroxide hydrate. When the reaction is carried out in the presence of an alkanol, the alkanol may be used as the solvent. The temperature at which the reaction is performed is preferably from about 0 to 100°C, more preferably 0 to 50°C, eg room temperature.

Compounds of formula II in which one or both of Y and Z represents C-$R_2$ in which $R_2$ represents alkyl C 1 to C6 may be made by alkylation of the corresponding compound in which Y or Z represents C-H. The reaction is preferably carried out under Friedel-Crafts conditions, ie

using $AlCl_3$ in an inert solvent, eg dichloromethane, at a temperature of from about -100 to $0^oC$.

Compounds of formula III in which W represents CO may be made from the corresponding carboxylic acid of formula III in which $L_a$ represents OH by conventional techniques known per se.

Compounds of formula VII may be prepared by reacting a compound of formula XXIII,

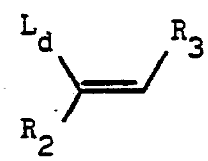

XXIII

in which $R_2$, $R_3$ and $L_d$ are as defined above, with a compound of formula XXIV,

$$R_4COL_2 \qquad XXIV$$

in which $R_4$ is as defined above and $L_2$ represents a leaving group.

The reaction is preferably carried out in the presence of a base catalyst, eg a metal alkoxide, such as sodium ethoxide, an amine, such as triethylamine, or a metal amide, such as lithium diisopropylamide. The reaction is preferably carried out in a solvent which is

 

inert to the reaction conditions. Good leaving groups that $L_2$ may represent include alkylamino, eg $-NHCH_3$.

The compounds of formula XII may be made from the corresponding carboxylic acid of formula XII in which $L_g$ represents OH by conventional techniques known *per se*. The carboxylic acid derivatives may be made from the corresponding carboxylic acid ester derivative.

Compounds of formula XII, in which $COL_g$ represents a carboxylic acid ester and $R_4$ represents $Ar_1-B-W$ or $Ar_1-B_b-CH_2$ may be prepared from the corresponding compound of formula II by a process analogous to process a) or process b) respectively.

Compounds of formula XIV may be prepared by nitration of a compound of formula XXV,

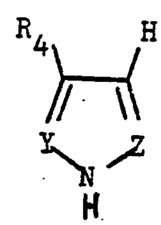

XXV

in which Y, Z and $R_4$ are as defined above.

The reaction is preferably carried out using acetyl nitrate in a polar solvent, eg acetic acid.

Compounds of formula XV in which $R_4$ represents $Ar_1-B-CO-$, may be prepared by oxidation of the corresponding compound in which $R_4$ represents

$Ar_1$-B-CHOH-, which in turn may be made by reaction of a compound of formula XXVI,

$Ar_1$-B-CHO XXVI

with a compound of formula XXVII,

$CH \equiv C-R_3$ XXVII

The reaction is preferably carried out in the presence of base in an inert solvent. The temperature at which the reaction is performed is preferably from about -150 to $0^oC$.

Compounds of formula XVIII may be prepared by reacting a compound of formula XXVIII,

$R_4$-CHO XXVIII

in which $R_4$ is as defined above,
with a compound of formula XXIX,

$Y_kH_2-L_k$ XXIX

in which $Y_k$ and $L_k$ are as defined above. This reaction is preferably performed in situ in process k).

Compounds of formulae IV - VI, VIII - XIV, XVI - XIX and XXI - XXIX are either known or can be prepared from known compounds by conventional methods known per se.

Certain compounds of formula I in which Y represents N and $R_1$ represents hydrogen may exist in a tautomeric form, formula A,

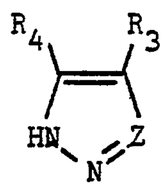

A

Similarly, certain compounds of formula I in which Z represents N and $R_1$ represents hydrogen may exist in a tautomeric form, formula B,

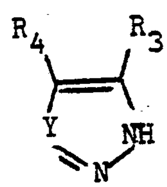

B

Compounds of formulae A and B are included within the scope of the definition of the compounds of formula I.

When any of the starting materials or intermediates contain a chiral centre they may be resolved using conventional techniques.

The processes as described above may produce the

compound of formula I or a derivative thereof. It is also within the scope of this invention to treat any derivative so produced to liberate the free compound of formula I, or to convert one derivative into another.

The compounds of formula I and the intermediates thereto may be isolated from their reaction mixtures using conventional techniques.

Pharmaceutically acceptable derivatives of those compounds of formula I in which $R_3$ represents -COOH include alkali metal salts such as sodium salts.

Pharmaceutically acceptable derivatives of the compounds of formula I also include compounds in which $R_1$ is replaced, for example by $-CH_2OCOR_{20}$, wherein $R_{20}$ represents alkyl, aryl or $N(alkyl)_2$.

Particular alkyl groups that $R_1$ may represent include methyl, ethyl, n-propyl and iso-propyl.

Particular alkyl groups that $R_2$ may represent include methyl, ethyl, 1-methylethyl, 1,1-dimethylethyl, n-propyl, n-butyl and 2-methylpropyl.

Particular alkyl groups that $R_{21}$ may represent include methyl, ethyl and 1-methylethyl.

Particular alkyl groups that $R_5$ may represent include methyl, ethyl and 1-methylethyl.

Particular groups that $R_6$ and $R_7$ may represent include hydrogen, methyl, phenyl, chlorophenyl, eg

2-chlorophenyl, (trifluoromethyl)phenyl, eg 2-(trifluoromethyl)phenyl, and phenylmethyl.

Particular alkyl groups that $R_8$ may represent include methyl, ethyl and 1-methylethyl.

Particular alkyl groups that $R_9$ may represent include methyl, ethyl, 1-methylethyl and propyl. Aryl groups which may be present as substituents on $R_9$ include aryl groups containing up to 6 ring atoms including 0, 1 or 2 heteroatoms. Preferred carbocyclic groups include phenyl. Preferred heterocyclic groups include those having 5 or 6 ring atoms, eg pyridyl and pyrryl. We particularly prefer the aryl group to be 2-pyridyl.

Particular halogens that $R_3$ may represent include fluorine, chlorine, bromine and iodine.

Preferred groups that $Ar_1$ may represent include phenyl; chlorophenyl, eg 2-chlorophenyl; dichlorophenyl, eg 2,3-, 2,4-, 2,5- and 2,6-dichlorophenyl; methylphenyl, eg 2-methylphenyl; (trifluoromethyl)phenyl, eg 2-(trifluoromethyl)phenyl; nitrophenyl, eg 2-nitrophenyl; aminophenyl, eg 2-aminophenyl; methoxyphenyl, 2-methoxyphenyl; hydroxyphenyl, eg 2-hydroxyphenyl; bromophenyl, eg 2-bromophenyl; iodophenyl, eg 2-iodophenyl; fluorophenyl, eg 2-fluorophenyl; pentafluorophenyl; 3,6-difluoro-2-(trifluoromethyl)phenyl;

2-chloro-5-nitrophenyl; naphthalenyl, eg 1-naphthalenyl; benzofurazanyl; and phenyl substituted by $-SCH_2Ar_2$, $-OCH_2Ar_2$ or $-OCOAr_2$ wherein $Ar_2$ is preferably phenyl or phenyl substituted by hydroxy, eg 2-hydroxyphenyl.

B preferably represents a single bond, $-CH_2-$ or $-CH_2-$ substituted by alkyl C 1 to C6, eg $-CH(CH_3)-$.

As a specific group of compounds we provide compounds of formula I in which both Y and Z represent $C-R_2$. We prefer the groups $R_2$ to be selected from methyl, ethyl, 1-methylethyl and 1,1-dimethylethyl.

As a further specific group we provide compounds of formula I in which $R_4$ represents $Ar_1CO-$. We prefer $Ar_1$ to be phenyl or phenyl substituted by one or more of X, $CX_3$, $-OCH_2Ar_2$ and $-OCOAr_2$, in which X represents chlorine, fluorine, bromine or iodine.

We prefer compounds of formula I in which $R_3$ represents $NO_2$ or $COOR_9$. We particularly prefer $R_9$ to be selected from the group methyl, ethyl, 1-methylethyl and 2-(2-pyridyl)ethyl.

As a particularly preferred group we provide compounds of formula I in which $R_3$ represents $COOR_9$ in which $R_9$ is selected from the group methyl, ethyl and 1-methylethyl, $R_4$ represents $Ar_1CO-$ in which $Ar_1$ represents phenyl or phenyl singly or multiply substituted

by one or more of X, $CX_3$, $-OCH_2Ar_2$ and $OCOAr_2$ in which X represents halogen and $Ar_2$ represents phenyl optionally substituted by hydroxy, and both Y and Z represent $C-R_2$ in which the groups $R_2$ are selected from the group methyl, ethyl and 1-methylethyl.

The compounds of formula I and pharmaceutically acceptable derivatives thereof are useful because they possess pharmacological activity in animals. In particular, the compounds are positive inotropic agents and are able to augment contractility of smooth and cardiac muscle. As such the compounds are suitable as cardiotonics.

The compounds of the invention are also able to increase the influx of $Ca^{++}$ into the cell and are thus suitable for the treatment of hypotonic circulatory conditions, for the depression of blood sugar, for decreasing the swelling of mucous membranes and for influencing the salt and fluid balance. The compounds may also be beneficial in the treatment of psychiatric illness, migraine and depression.

Activity of the compound has been observed in the following assay systems:

1. Guinea Pig Atria: Force and Rate - R M Kennedy and E Seifer, Eur. J. Pharmac. (1985), 107, 209-14;

2. Accumulation of $^{45}Ca$ in GH3 cells: G A Shangold,

S Kongsamnt and R J Miller, Life Sciences (1985), 36,2209-15.

The dosage administered will naturally depend on the compound employed, the mode of administration and the desired effect. However in general satisfactory results are obtained when the compounds are administered at a dosage of from 0.05µg to 3.5g, which may be administered in divided doses of, for example 1µg to 750mg.

The compounds of formula I, and pharmaceutically acceptable derivatives thereof, have the advantage that they are more efficacious or produce less undesirable side effects in certain pharmacological models, or are longer acting than compounds of similar structure to the compounds of formula I.

The compounds of the invention may be administered by a wide variety of routes and may act systemically or locally. Thus the compounds may be administered by oral or nasal inhalation to the lung, to the buccal cavity, oesophageally, rectally, topically to the skin or to other available surfaces of the body by injection, eg intravenously, intramuscularly, intraperitoneally, or by surgical implant.

According to our invention we also provide a pharmaceutical composition comprising preferably less than 80% and more preferably less than 50% by weight of a

compound of formula I, or a pharmaceutically acceptable derivative thereof, in admixture with a pharmaceutically acceptable adjuvant, diluent or carrier. Examples of suitable adjuvants, diluents or carriers are: for tablets, capsules and dragees; microcrystalline cellulose, calcium phosphate, diatomaceous earth, a sugar such as lactose, dextrose or mannitol, talc. stearic acid, starch, sodium bicarbonate and/or gelatin;

for suppositories; natural or hardened oil or waxes; and

for inhalation compositions; coarse lactose.

When the compounds are to be used in aqueous solution it may be necessary to incorporate a chelating or sequestering agent, eg sodium edetate, an antioxidant, eg sodium metabisulphite or buffering agents, eg sodium hydrogen phosphate and sodium phosphate. Aqueous solutions typically contain up to about 10% w/w of the new compound and may be used for intravenous injections.

According to the invention, we further provide a method of increasing the force of contraction of the heart in an animal, either human or non-human, which method comprises administering to the animal an effective amount of one or more compounds of the invention.

The invention is illustrated, but in no way limited by the following Examples. All temperatures quoted are in

°C.

Example 1

Methyl 4-(2-Chlorobenzoyl)-2,5-dimethyl-1H-pyrrole-3-carboxylate

A solution of methyl 2,5-dimethyl-1H-pyrrole-3-carboxylate (2.78g, 18mmoles) in dry dichloromethane (20ml) was added, dropwise, to a suspension of aluminium chloride (6.40g, 50mmoles) in dry dichloromethane (40ml), the temperature being maintained at 0°. Stirring was continued for 10 minutes after which time a solution of 2-chlorobenzoyl chloride (3.50g, 0.02mmoles) in dry dichloromethane (20ml) was added, dropwise, at 0°. The reaction mixture was warmed to room temperature over a period of 1 hour and stirred for a further 16 hours.

The reaction mixture was poured onto a mixture of ice (200ml) and concentrated HCl (20ml). The dichloromethane layer was separated and the aqueous layer extracted with dichloromethane (2 x 50ml). The combined organic extracts were washed with 2N HCl (2 x 50ml), water and saturated aqueous sodium bicarbonate solution, dried ($Na_2SO_4$) and concentrated, in vacuo, to give a brown oily solid. This solid was chromatographed on silica gel using ethyl acetate/petroleum ether mixtures as eluant and gave a yellow solid (4.7g) that was recrystallised from toluene to give the title product (3.75g), mp 129.0-129.5°.

Example 2

Methyl 2,5-Dimethyl-4-(2-(trifluoromethyl)benzoyl)-1H-pyrrole-3-carboxylate

Methyl 2,5-dimethyl-1H-pyrrole-3-carboxylate (2.8g, 18mmoles) in dichloromethane (15ml) was added to aluminium trichloride (7g) in dichloromethane (200ml) at -78°. After heating up to 0° slowly, the mixture was recooled to -78° and 2-(trifluoromethyl)benzoyl chloride (4g, 18mmoles) in dichloromethane (20ml) was added. After 40 minutes at -10°, the reaction mixture was poured onto ice and chloroform. The organic layer was separated, dried ($Na_2SO_4$) and the residue chromatographed on silica eluting with ethyl acetate/hexane mixtures. Trituration with ether gave the title compound (1.7g), mp 166-167°.

The compounds of Examples 3 to 16 inclusive were made by methods analogous to Examples 1 and 2 using the appropriate starting materials.

Example 3

Methyl 4-Benzoyl-2,5-dimethyl-1H-pyrrole-3-carboxylate

mp 114-115°

Example 4

Methyl 2,5-Dimethyl-4-(2-nitrobenzoyl)-1H-pyrrole-3-carboxylate

0230110

mp 121-122°

Example 5

Methyl 4-(2-Methoxybenzoyl)-2,5-dimethyl-1H-pyrrole-3-carboxylate

mp 117-118°

Example 6

Methyl 2,5-Dimethyl-4-(1-naphthalenylcarbonyl)-1H-pyrrole-3-carboxylate

mp 154-155°

Example 7

Methyl 2,5-Dimethyl-4-(2-methylbenzoyl)-1H-pyrrole-3-carboxylate

mp 112-113°

Example 8

Methyl 4-((2-Chlorophenyl)acetyl)-2,5-dimethyl-1H-pyrrole-3-carboxylate

mp 125.0-125.5°

Example 9

Methyl 4-(4-Benzofurazanylcarbonyl)-2,5-dimethyl-1H-pyrrole-3-carboxylate

a) 4-Benzofurazancarbonyl chloride

Oxalyl chloride (3ml) was added to 4-benzofurazancarboxylic acid (3g, 18.3mmoles) in 1,2-dichloroethane (60ml). The mixture was heated at reflux for 3 hours and the solvent was evaporated to give the sub-title acid

chloride.

b) Methyl 4-(4-Benzofurazanylcarbonyl)-2,5-dimethyl-1H-pyrrole-3-carboxylate

Prepared by the method of Example 1 using the product of step a).

mp 202-203°.

Example 10

Methyl 4-(2,5-Dichlorobenzoyl)-2,5-dimethyl-1H-pyrrole-3-carboxylate

mp 169-170°.

Example 11

Methyl 4-(2,3-Dichlorobenzoyl)-2,5-dimethyl-1H-pyrrole-3-carboxylate

mp 133-134°

Example 12

Methyl 4-(3,6-Difluoro-2-(trifluoromethyl)benzoyl)-2,5-dimethyl-1H-pyrrole-3-carboxylate

a) 3,6-Difluoro-2-(trifluoromethyl)benzoic acid

Jones reagent was added to 3,6-difluoro-2-(trifluoromethyl)benzaldehyde (30g, 0.143mole) in acetone (200ml) at 0°. After stirring for 2 hours at room temperature, the solvent was evaporated and the residue was dissolved in chloroform and water. The organic layer was separated, dried ($MgSO_4$) and the solvent was evaporated. Recrystallisation from carbon tetrachloride

gave the sub-title acid (27g).

$M^+$ 226, nmr ($CDCl_3$) delta 7.4-7.25 (m,2H).

b) 3,6-Difluoro-2-(trifluoromethyl)benzoyl chloride

Prepared by the method of Example 9a) from the product of step a).

c) Methyl 4-(3,6-Difluoro-2-(trifluoromethyl)benzoyl)-2,5-dimethyl-1H-pyrrole-3-carboxylate

Prepared by the method of Example 1 using the product of step b).

mp 152-154°

Example 13

Methyl 4-(2,4-Dichlorobenzoyl)-2,5-dimethyl-1H-pyrrole-3-carboxylate

mp 130-131°

Example 14

Methyl 4-(2,6-Dichlorobenzoyl)-2,5-dimethyl-1H-pyrrole-3-carboxylate

mp 190-191°

Example 15

Methyl 4-(2-Fluorobenzoyl)-2,5-dimethyl-1H-pyrrole-3-carboxylate

mp 131-132°

Example 16

Methyl 2,5-Dimethyl-4-(pentafluorobenzoyl)-1H-pyrrole-3-carboxylate

mp 168-169°

Example 17

Methyl 4-(2-(2-Hydroxybenzoyloxy)benzoyl)-2,5-dimethyl-1H-pyrrole-3-carboxylate

Methyl 2,5-dimethyl-1H-pyrrole-3-carboxylate (2.5g, 18mmoles) in dichloromethane (30ml) was added to a stirred suspension at -78° of aluminium chloride (7.5g) in dichloromethane (140ml). The reaction mixture was warmed to 0° and then recooled to -78°. 2-Hydroxybenzoyl chloride (2.7g, 18mmoles) in dichloromethane (10ml) was added and the reaction mixture allowed to warm up to -30°. The reaction mixture was poured onto ice and the product extracted out with chloroform. The organic extract was dried ($MgSO_4$) and the solvent was evaporated. Chromatography (x2) on silica eluting with ethyl acetate/hexane mixtures followed by trituration with ether gave the title compound (0.32g), mp 172-173°.

The compounds of Examples 18 to 26 inclusive were prepared by methods analogous to Examples 1 and 2 using the appropriate starting materials.

Example 18

Methyl 4-(2-Iodobenzoyl)-2,5-dimethyl-1H-pyrrole-3-carboxylate

mp 125-126°

Example 19

Methyl 4-((2-Chlorophenyl)thio)-2,5-dimethyl-1H-pyrrole-3-carboxylate

Prepared using 2-chlorobenzenesulphenyl chloride without catalyst, mp 200-201°.

Example 20

1-(4-(2-Chlorobenzoyl)-2,5-dimethyl-1H-pyrrol-3-yl)ethanone

mp 211-212°

Example 21

Methyl 4-(2-Bromobenzoyl)-2,5-dimethyl-1H-pyrrole-3-carboxylate

mp 113-115°

Example 22

Methyl 4-(2-Chlorobenzoyl)-5-ethyl-2-methyl-1H-pyrrole-3-carboxylate

mp 121-122°

Example 23

Methyl 4-(2-Chlorobenzoyl)-2-methyl-5-(1-methylethyl)-1H-pyrrole-3-carboxylate

a) Methyl 2-Methyl-5-(1-methylethyl)-1H-pyrrole-3-carboxylate

Methyl 2-Methyl-1H-pyrrole-3-carboxylate (1g, 7.2mmoles) in dichloromethane (10ml) was added to a stirred suspension at -78° of aluminium chloride (2.9g,

21.6mmoles) and dichloromethane (45ml). The reaction was warmed to room temperature and then recooled to -78°. 2-Chloropropane (0.6g, 7.2mmoles) in dichloromethane (10ml) was added and the reaction allowed to reach room temperature. The reaction mixture was poured onto ice and the product was extracted with chloroform (x2). The combined organic extracts were dried ($MgSO_4$) and the solvent was evaporated. Chromatography on silica eluting with ethyl acetate/dichloromethane mixtures give the sub-title compound (0.44g).

$M^+$ 195; nmr ($CDCl_3$) delta 3.78 (s,3H), 2.47 (s,3H) and 1.23 (d,6H).

b) Methyl 4-(2-Chlorobenzoyl)-2-methyl-5-(1-methylethyl)-1H-pyrrole-3-carboxylate

Prepared by the method of Example 1 using the product of step a).

mp 119-120°.

Example 24

Methyl 4-(2-Chlorobenzoyl)-5-(1,1-dimethylethyl)-2-methyl-1H-pyrrole-3-carboxylate

Reaction time - 3 hours at room temperature.

$M^+$ 333/5; nmr ($CDCl_3$) delta 3.38 (s,3H), 2.46 (s,3H) and 137 (s,9H).

Example 25

Methyl 2,5-Dimethyl-4-(2-((phenylmethyl)thio)benzoyl)-

1H-pyrrole-3-carboxylate

a) 2-((Phenylmethyl)thio)benzoyl chloride

Thionyl chloride (0.6g, 5.5mmoles) was added to a mixture of 2-((phenylmethyl)thio)benzoic acid (1.22g, 5mmoles) and dichloromethane (20ml). The reaction mixture was heated at reflux for 2 hours and then the solvent was evaporated to give the sub-title acid chloride.

b) Methyl 2,5-Dimethyl-4-(2-((phenylmethyl)thio)benzoyl)-1H-pyrrole-3-carboxylate

Prepared by the method of Example 1 using the product of step a).

$M^+$ 379; nmr ($CDCl_3$) delta 4.18 (s,2H), 3.10 (s,3H), 2.42 (s,3H) and 2.25 (s,3H).

Example 26

Methyl 4-(2-Chloro-5-nitrobenzoyl)-2,5-dimethyl-1H-pyrrole-3-carboxylate

$M^+$ 336/8; nmr ($CDCl_3$) delta 3.34 (s,3H), 2.45 (s,3H) and 2.44 (s,3H).

Example 27

Methyl 4-(2-Carboxybenzoyl)-2,5-dimethyl-1H-pyrrole-3-carboxylate

Prepared by the method of Example 1 using 1,3-isobenzofurandione. $M^+$ 301; NMR ($CDCl_3$) delta 3.25 (s,3H), 2.38 (s,3H), 2.27 (s,3H).

Example 28

Ethyl 4-((2-Chlorophenyl)methyl)-2,5-dimethyl-1H-pyrrole-3-carboxylate

A mixture of hydroiodic acid (10ml of 51%) and hypophosphorous acid (10ml) was shaken until all the brown colour had been removed and then added to a solution of ethyl 2,5-dimethyl-1H-pyrrole-3-carboxylate (1.67g, 11 mmoles) and 2-chlorobenzaldehyde (1.69g, 12 mmoles) in acetic acid (30ml). After stirring for 2 hours at room temperature under $N_2$, the reaction mixture was poured onto water (200ml) and extracted with ether (3 x 50ml). The combined extracts were washed with water, saturated sodium bicarbonate, water and brine, dried ($Na_2SO_4$) and the solvent was evaporated. Chromatography of the residue on silica eluting with chloroform, followed by recrystallisation from toluene gave the title compound (0.4g), mp 157.5-159°

Example 29

2-Methyl-N-phenyl-4-(2-(trifluoromethyl)phenyl)-1H-pyrrole-3-carboxamide

A mixture of 2-amino-1-(2-(trifluoromethyl)phenyl)-ethanone hydrochloride (7.19g, 0.03mmoles, prepared using the method described in J. Org. Chem. 1977, 42, 870) and N-phenyl-3-oxobutanamide (5.31g, 0.03mmoles) in warm 2-propanol (200ml) was added to a sodium acetate buffer (300ml, pH 4) and the mixture heated to reflux for three

days. The majority of the 2-propanol was removed *in vacuo*. The residue was diluted with water (200ml) and the aqueous suspension extracted with ethyl acetate (3x100ml). The combined extracts were washed with water, saturated sodium bicarbonate solution and brine, dried ($Na_2SO_4$) and evaporated. The residue was chromatographed on silica gel using dichloromethane as eluant and the product recrystallised from a mixture of toluene and petroleum ether (bp 60-80°) to give the title product (0.85g), mp 162-3°

The compounds of Examples 30 - 33 were prepared by the method of Example 29 using the appropriate starting materials.

Example 30

Ethyl 4-(2-Chlorophenyl)-2-methyl-1H-pyrrole-3-carboxylate

mp 146-147°

Example 31

Methyl 2-Methyl-4-(2-(trifluoromethyl)phenyl)-1H-pyrrole-3-carboxylate

mp 163-164.5°

Example 32

4-(2-Chlorophenyl)-2-methyl-N-phenyl-1H-pyrrole-3-carboxamide

mp 158.5-159.5°

0230110

Example 33

4-(2-Chlorophenyl)-2-methyl-3-nitro-1H-pyrrole

mp 207-209°

Example 34

Methyl 2,5-Dimethyl-4-(1-phenylethyl)-1H-pyrrole-3-carboxylate

Methyl 3-amino-2-butenoate (11.5g, 0.1moles), α- methylbenzeneacetaldehyde (13.4g, 0.1moles) and nitroethane (7.5g, 0.1moles) were heated at reflux in t-butanol (50ml) for 4 days. The solvent was removed and the residue purified by HPLC on silica eluting with ethyl acetate/petroleum ether (60-80°) mixtures. Crystallisation from ether/hexane gave a solid that was further purified by chromatography on silica eluting with ether and crystallisation from ether/hexane to give the title compound (0.26g), mp 137-138°.

Example 35

Methyl 2,5-Dimethyl-4-(1-naphthalenyl)-1H-pyrrole-3-carboxylate

3-Naphthalenyl-2-nitroprop-2-ene (4.26g, 20mmoles) and methyl 3-amino-2-butenoate (2.3g, 20mmoles) were heated at reflux for 4 days in methanol. The reaction mixture was then partitioned between water and ethyl acetate and the organic layer separated and dried ($Na_2SO_4$). Chromatography on silica eluting with ethyl

0230110

acetate/hexane mixtures gave the title compound (0.85g), mp 177-178°.

Example 36

Methyl 4-(2-Chlorobenzoyl)-5-methyl-1H-pyrrole-3-carboxylate

a) (2-(2-Chlorophenyl)-2-oxoethyl)triphenylphosphonium bromide

2-Bromo-1-(2-chlorophenyl)ethanone (50g, 0.214moles) in toluene (50ml) was added dropwise to a solution of triphenylphosphine (56g, 0.214moles) in toluene (300ml). After stirring for 1 day, the product was filtered, washed with ether and dried to give the sub-title salt (100g).

$M^+$ 415/7.

b) Methyl 4-(2-chlorophenyl)-4-oxo-2-butenoate

Butyl lithium (90ml of 1.5M in hexane, 0.135mol) was added to a solution at -78° of (2-(2-chlorophenyl)-2-oxoethyl)triphenylphosphonium bromide (65g, 0.13mol) in tetrahydrofuran (500ml). After warming to room temperature, methyl glyoxalate (10g, 0.114mole) in tetrahydrofuran (40ml) was added. After stirring for 16 hours, the solvent was evaporated and ether and water were added. The organic layer was separated, dried ($MgSO_4$) and the solvent was evaporated. Chromatography on silica eluting with ethyl acetate/petroleum ether (60-80°) mixtures gave the sub-title ester (15.4g).

$M^+$224/6; nmr ($CDCl_3$) delta 7.54 (d,1H), 6.67 (d,1H) and 3.83 (s,3H).

c) Methyl 4-(2-Chlorobenzoyl)-5-methyl-1H-pyrrole-3-carboxylate

Methyl 4-(2-chlorophenyl)-4-oxo-2-butenoate (1.64g, 7.2mmoles) and 1-((1-isocyanoethyl)sulphonyl)-4-methylbenzene (1.5g, 7.2mmoles) in ether/dimethylsulphoxide (50ml of 2:1) was added dropwise to a stirred mixture at -5° of sodium hydride (0.21g, 8.6mmoles) in ether (15ml). After 2 hours at room temperature, the reaction mixture was poured onto ethyl acetate/water/brine. The organic layer was separated and the aqueous layer extracted with ethyl acetate. The combined organic extracts were washed with water, dried ($MgSO_4$) and the solvent was evaporated. Chromatography on silica eluting with ethyl acetate/dichloromethane mixtures followed by trituration with ether/cyclohexane gave the title compound (0.134g), mp 111-112°.

Example 37

Methyl 3-(2-Chlorophenyl)-5-methyl-1H-pyrazole-4-carboxylate

a) Methyl 2-(2-chlorobenzoyl)-3-(methylamino)-2-butenoate

2-Chlorobenzoyl chloride (2.8g, 11.6mmol) in toluene (5ml) was added dropwise to a solution at 0° of methyl 3-(methylamino)-2-butenoate (2g, 15.5mmol) and

triethylamine (2.36, 23.3mmol) in toluene (50ml). After stirring for 16 hours at room temperature, water and ether were added. The ethereal layer was separated, dried ($Na_2SO_4$) and the solvent evaporated. Chromatography on silica, eluting with ethyl acetate/hexane gave the sub-title compound (2.9g).

$M^+$ 267/9; nmr delta ($CDCl_3$) 3.25 (s,3H), 2.35 (s,3H).

b) Methyl 3-(2-Chlorophenyl)-5-methyl-1H-pyrazole-4-carboxylate

Hydrazine hydrate (0.6g, 13mmoles) was added to a stirred solution of methyl 2-(2-chlorobenzoyl)-3-(methylamino)-2-butenoate (3.1g, 11.6mmoles) in acetic acid (30ml). After 2 hours at room temperature, the mixture was heated at reflux for 1 hour. The solvent was evaporated and the residue dissolved in chloroform. This solution was washed with water, dilute aqueous ammonia and water, dried ($Na_2SO_4$) and the solvent was evaporated. Chromatography on silica eluting with ethyl acetate/hexane mixtures gave the title compound (0.5g), mp 119-120°.

Example 38

Methyl 3-((2-Chlorophenyl)methyl)-5-methyl-1H-pyrazole-4-carboxylate

a) Methyl 2-((2-chlorophenyl)acetyl)-3-(methylamino)-2-butenoate

Prepared by the method of Example 37a).

$M^+$ 281/3; nmr ($CDCl_3$) delta 4.0 (s,2H) and 3.68 (s,3H).

b) Methyl 3-((2-Chlorophenyl)methyl)-5-methyl-1H-pyrazole-4-carboxylate

Prepared by the method of Example 37b) using methyl 2-((2-chlorophenyl)acetyl)-3-(methylamino)-2-butenoate.

mp 135-136°.

Example 39

i) Methyl 4-(2-Chlorobenzoyl)-5-methyl-1H-pyrazole-3-carboxylate

ii) Methyl 3-(2-Chlorobenzoyl)-5-methyl-1H-pyrazole-4-carboxylate

a) Methyl 4-(2-chlorophenyl)-4-hydroxy-2-butyne carboxylate

Butyl lithium (1.55M in hexane, 15.5ml, 25mmoles) was added quickly to a solution at -130° of methyl propynoate (2.1g, 25mmoles) in tetrahydrofuran/ether/pentane (4:1:1, 100ml). 2-Chlorobenzaldehyde (3.5g, 25mmoles) was added almost immediately and the reaction warmed up slowly to -80°. After 1 hour, chlorotrimethylsilane (2ml) in ether (10ml) was added, the reaction warmed to -40° and then poured onto water/ethyl acetate. The organic layer was separated, washed with water, 1% hydrochloric acid, water and brine, dried

($MgSO_4$) and the solvent was evaporated. The sub-title compound was obtained as a pale yellow oil (5.2g).

b) <u>Methyl 4-(2-chlorophenyl)-4-oxo-2-butynecarboxylate</u>

Jones reagent (75ml) was added dropwise to a solution maintained at 5-10° of methyl 4-(2-chlorophenyl)-4-hydroxy-2-butynecarboxylate (21.5g) in acetone (200ml). After 45 minutes at 15-20°, the reaction mixture was cooled to 0° and treated dropwise with ethanol (40ml). Water was added and this solution was extracted with ethyl acetate (x3). The combined extracts were washed with water, brine and dried ($MgSO_4$). Evaporation of solvent followed by purification by HPLC on silica eluting with ethyl acetate/petroleum ether (60-80°) mixtures gave the sub-title compound (21.5g).

Nmr ($CDCl_3$) delta 3.88 (s,3H).

c) <u>Methyl 4-(2-Chlorobenzoyl)-5-methyl-1H-pyrazole-3-carboxylate and Methyl 3-(2-Chlorobenzoyl)-5-methyl-1H-pyrazole-4-carboxylate</u>

Methyl 4-(2-chlorophenyl)-4-oxo-2-butynecarboxylate (40g, 18mmoles) in ether (80ml) was treated at -20° with excess diazoethane. After 1 hour, the reaction mixture was warmed to room temperature and the solvent was evaporated. Chromatography on silica eluting with ethyl acetate gave the pyrazole-3-carboxylate (0.27g), mp 95-96° and the pyrazole-4-carboxylate (3.2g),

mp 97-98°.

Example 40

Methyl 3-(Chlorobenzoyl)-1H-pyrazole-4-carboxylate

Prepared by the method of Example 39, mp 189-191°.

Example 41

i) 3-Ethyl 5-Methyl 4-(2-Chlorobenzoyl)-1H-pyrazole-3,5-dicarboxylate

ii) 3-Ethyl 4-Methyl 5-(2-Chlorobenzoyl)-1H-pyrazole-3,4-dicarboxylate

Prepared by the method of Example 39.

Pyrazole-3,5-dicarboxylate: $M^+$ 336/8; NMR ($CDCl_3$) delta 3.82 (s,3H), 1.15 (t,3H).

Pyrazole-3,4-dicarboxylate: $M^+$ 336/8; NMR ($CDCl_3$) delta 3.81 (s,3H), 1.14 (t,3H).

Example 42

Methyl 4-(2-Aminobenzoyl)-2,5-dimethyl-1H-pyrrole-3-carboxylate

Methyl 2,5-dimethyl-4-(2-nitrobenzoyl)-1H-pyrrole-3-carboxylate (1g, 3.3mmoles) in methanol (50ml) was hydrogenated at 1 atmosphere over platinum oxide. On completion, the catalyst was filtered off and the solvent was evaporated. Chromatography on silica eluting with ether followed by rechromatography on silica eluting with dichloromethane/methanol mixtures and trituration with ether gave the title compound (0.15g), mp 73-76°.

Example 43

4-(2-Chlorobenzoyl)-2,5-dimethyl-1H-pyrrole-3-carboxylic acid

Trimethylsilyl chloride (39.2g, 0.36mmoles) was added to a stirred mixture of methyl 4-(2-chlorobenzoyl)-2,5-dimethyl-1H-pyrrole-3-carboxylate (35.4g, 0.13mmoles) and sodium iodide (54g, 0.36mmoles) in acetonitrile (250ml). After heating at reflux for 2 hours, most of the solvent was evaporated and the residue mixed with 2N sulphuric acid (1000ml). The product was extracted with ethyl acetate (x3) and the combined extracts were washed with water (x2), aqueous sodium thiosulphate (x2), water and brine. After drying ($MgSO_4$) and removal of solvent, the residue was recrystallised from ethanol to give the title acid (4.67g), mp 230-231$^{o}$.

Example 44

1-Methylethyl 4-(2-Chlorobenzoyl)-2,5-dimethyl-1H-pyrrole-3-carboxylate

4-(2-Chlorobenzoyl)-2,5-dimethyl-1H-pyrrole-3-carboxylic acid (2g, 7.2mmoles) was dissolved in thionyl chloride (10ml) and dimethylformamide (1 drop). After 3 hours, the solvent was evaporated and the residue dissolved in 2-propanol (50ml). After 16 hours, the solvent was evaporated and the residue dissolved in ethyl acetate/water. The organic layer was separated, washed

with saturated sodium bicarbonate and brine, dried ($Na_2SO_4$) and the solvent was evaporated. Chromatography on silica eluting with ethyl acetate/ petroleum ether (60-80°) mixtures followed by rechromatography on silica eluting with ether and crystallisation from 2-propanol/hexane gave the title compound (0.4g), mp 103-105°.

<u>Example 45</u>

<u>2-(2-Pyridinyl)ethyl) 4-(2-Chlorobenzoyl)-2,5-dimethyl-1H-pyrrole-3-carboxylate</u>

Prepared by the method of Example 44, mp 139-139.5°.

<u>Example 46</u>

<u>4-(2-Chlorobenzoyl)-2,5-dimethyl-N-phenyl-1H-pyrrole-3-carboxamide</u>

Prepared by the method of Example 44, mp 237-238°.

<u>Example 47</u>

<u>4-(2-Chlorobenzoyl)-2,5-dimethyl-N-(phenylmethyl)-1H-pyrrole-3-carboxamide</u>

Triphenylphosphine (0.79g, 3mmoles) in acetonitrile (2ml) and pyridine (2ml) was added to a stirred mixture of 4-(2-chlorobenzoyl)-2,5-dimethyl-1H-pyrrole-3-carboxylic acid (0.54g, 2mmoles), phenylmethylamine (0.21g, 2mmoles), triethylamine (0.6g, 6mmoles) in acetonitrile (5ml) and pyridine (5ml). After stirring for 16 hours, the solvent was evaporated and the residue dissolved in

dichloromethane. This organic solution was washed with dilute hydrochloric acid, water, saturated sodium bicarbonate and brine, dried ($MgSO_4$) and the solvent was evaporated. Chromatography on silica eluting with ethyl acetate gave the title compound (0.27g).

$M^+$ 366/8; nmr ($CDCl_3$) delta 4.57 (s,2H), 2.22 (s,3H) and 1.68 (s,3H).

Example 48

i) Methyl 4-(2-Chlorobenzoyl)-5-(hydroxymethyl)-2-methyl -1H-pyrrole-3-carboxylate

ii) Methyl 4-(2-Chlorobenzoyl)-5-(methoxymethyl)-2-methyl -1H-pyrrole-3-carboxylate

Lithium hydroxide hydrate (8.4mg, 0.2mmoles) was added to methyl 5-(acetoxymethyl)-4-(2-chlorobenzoyl)-2-methyl -1H-pyrrole-3-carboxylate (35mg, 0.1mmoles) in methanol (0.5ml). After stirring for 16 hours, the reaction mixture was acidified with dilute hydrochloric acid and extracted with ethyl acetate. The organic extract was dried ($MgSO_4$) and the solvent was evaporated. Purification by preparative thin layer chromatography on silica eluting with ethyl acetate/petroleum ether (60-80$^o$) gave the hydroxymethyl compound (10mg), $M^+$, 307/9; nmr ($CDCl_3$) delta 4.68 (s,2H), 3.2 (s,3H) and 2.4 (s,3H) and the methoxymethyl compound (10mg), $M^+$, 321/3;

nmr ($CDCl_3$) delta 4.67 (s,2H), 3.45 (s,3H), 3.22 (s,3H) and 2.45 (s,3H).

Example 49

Methyl 1,2,5-trimethyl-4-(2-(trifluoromethyl) benzoyl)-1H-pyrrole-3-carboxylate

Methyl 2,5-dimethyl-4-[2-(trifluoromethyl)benzoyl]-1H-pyrrole-3-carboxylate (0.3g, 0.9mmoles) in tetrahydrofuran (1ml) was added to sodium hydride (35mg), warmed to 40$^{\circ}$ for 2 hours, then treated with methyl iodide (3ml) and refluxed for 3 hours. The mixture was evaporated, diluted with aqueous hydrochloric acid, extracted with chloroform, dried and evaporated. Chromatography on silica eluting with ethyl acetate/hexane mixtures gave the title compound (0.26g), mp 119-120$^{\circ}$.

Example 50

4-(2-Chlorobenzoyl)-2,5-dimethyl-1H-pyrrole-3-carboxaldehyde

(2-Chlorophenyl)(2,5-dimethyl-1H-pyrrol-3-yl)methanone (4.8g, 20.5mmoles) in dichloromethane was added to dichloromethane (200ml) containing dimethylformamide (10ml) and phosphorous oxychloride (2ml) at 0$^{\circ}$. After 4 hours at room temperature the reaction was added to ice, extracted with ether, dried ($MgSO_4$) and the solvent evaporated. Chromatography on silica eluting with ethyl acetate and dichloromethane gave the title compound

 

(0.37g), mp 180-181°.

Example 51

(4-Bromo-2,5-dimethyl-1H-pyrrol-3-yl)(2-chlorophenyl) methanone

(2-Chlorophenyl)(2,5-dimethyl-1H-pyrrol-3-yl)methanone (2g, 8.56mmoles) in chloroform (80ml) and triethylamine (10ml) was treated portionwise with pyridinium bromide perbromide (2.9g) at a temperature between 2 and 7°. The crude reaction mixture was then evaporated onto silica and eluted with ethyl acetate and hexane to give the title compound (2.52g), mp 159-160° (dec).

Example 52

4-Bromo-3-(2-chlorophenyl)-5-methyl-1H-pyrazole

Prepared by the method of Example 51. $M^+$ 272; NMR ($CDCl_3$) delta 2.11 (s,3H).

Example 53

Methyl 4-[(2-Chlorophenyl)sulphinyl]-2,5-dimethyl -1H-pyrrole-3-carboxylate

Methyl 4-((2-chlorophenyl)thio)-2,5-dimethyl-1H-pyrrole-3-carboxylate (0.9g, 3mmoles) in chloroform (30ml) was treated with 3-chlorobenzeneperoxoic acid (0.6g, 3.4mmoles)) in chloroform (20ml). The reaction was stirred for 1 hour at room temperature, quenched by addition of aqueous sodium bicarbonate and the organic layer was separated and dried ($Na_2SO_4$), and the

solvent evaporated. Chromatography on silica eluting with ethyl acetate/petroleum ether (60-80°) mixtures followed by trituration with cyclohexane gave the title compound (0.81g), mp 143-144° (dec).

Example 54

Methyl 4-((2-Chlorophenyl)sulphonyl)-2,5-dimethyl-1H-pyrrole-3-carboxylate

Prepared by the method of Example 53 using 3 molar equivalents of the oxidant, mp 191-192°.

Example 55

Methyl 4-[(2-Chlorophenyl)(hydroxyimino)methyl]-2,5-dimethyl-1H-pyrrole-3-carboxylate

Methyl 4-(2-chlorobenzoyl)-2,5-dimethyl-1H-pyrrole-3-carboxylate (0.7g, 2.4mmoles), hydroxylamine hydrochloride (0.7g) and pyridine (0.7ml) were refluxed in ethanol (14ml) for 12 hours. The residue was partitioned between aqueous hydrochloric acid and ethyl acetate, and the organic layer was separated, dried ($MgSO_4$) and evaporated. Chromatography on silica eluting with ethyl acetate and hexane mixtures followed by crystallisation from ether/cyclohexane gave the title compound (0.5g), mp 150-151°.

Example 56

(2-Chlorophenyl)(2,5-dimethyl-4-nitro-1H-pyrrol-3-yl) methanone

(2-Chlorophenyl)(2,5-dimethyl-1H-pyrrol-3-yl)methanone (1.66g, 7.1mmoles) in acetonitrile (10ml) was added to a solution at -45° of nitronium tetrafluoroborate (1.0g, 7.5mmoles) in acetonitrile (60ml). The reaction was warmed slowly to 10° and then left for 30 minutes. The reaction mixture was poured onto ice and 1% aqueous sodium bicarbonate and the product extracted with ethyl acetate (x2). The combined extracts were washed with water and brine, dried ($MgSO_4$) and the solvent was evaporated. Chromatography on silica eluting with ethyl acetate/dichloromethane mixtures followed by trituration with ether/hexane gave the title compound (0.46g), mp 148-150°.

Example 57

3-(2-Chlorophenyl)-5-methyl-4-nitro-1H-pyrazole

a) 3-(2-Chlorophenyl)-5-methyl-1-nitro-1H-pyrazole

3-(2-Chlorophenyl)-5-methyl-1H-pyrazole (2.3g, 11.95mmoles) was added to a stirred mixture of nitric acid (1.7ml, d 1.52) and acetic anhydride (4ml) in acetic acid (20ml). After 2 hours, the reaction mixture was poured onto ice/water and the solid filtered off. Recrystallisation from methanol gave the sub-title compound (1.4g), mp 94-95°.

b) 3-(2-Chlorophenyl)-5-methyl-4-nitro-1H-pyrazole

The product of step a) (1.0g, 4.2mmoles) was heated

at reflux in chlorobenzene (15ml) for 4 hours. The solvent was evapaorated and the residue chromatographed on silica eluting with ethyl acetate/hexane mixtures. Trituration with petroleum ether (60-80°) gave the title compound (0.18g), mp 125-126°.

Example 58

Methyl 4-(2-Chlorobenzoyl)-2-methyl-1H-pyrrole-3-carboxylate

Prepared by the method of Example 1 using methyl 5-(1,1-dimethylethyl)-2-methyl-1H-pyrrole-3-carboxylate and a reaction time of 3 days at room temperature, mp 174-175°.

Example 59

Methyl 4-(2-Hydroxybenzoyl)-2,5-dimethyl-1H-pyrrole-3-carboxylate

Boron trichloride (1ml of 1M solution in dichloromethane, 1mmole) was added to a stirred solution at -78° of methyl 4-(2-methoxybenzoyl)-2,5-dimethyl-1H-pyrrole-3-carboxylate (0.287g, 1mmole) in dichloromethane (5ml). The reaction mixture was warmed up to room temperature and stirred for 16 hours. Methanol was added and the resulting solution evaporated to dryness. Chromatography on silica eluting with ethyl acetate/hexane mixtures gave the title compound (0.1g).

$M^+$ 273; nmr ($CDCl_3$) delta 3.35 (s,3H), 2.43

(s,3H) and 2.13 (s,3H).

Example 60

Methyl 2,5-Dimethyl-4-(2-(phenylmethoxy)benzoyl)-1H-pyrrole-3-carboxylate

Sodium hydride (52mg of 80% in oil, 1.73mmoles) was added to a stirred solution of methyl 4-(2-hydroxybenzoyl)-2,5-dimethyl-1H-pyrrole-3-carboxylate (0.47g), 1.72mmoles) in dimethylformamide (5ml). Benzyl bromide (0.32g, 1.9mmoles) was added and the mixture stirred for 16 hours. The reaction mixture was poured onto ice and the product extracted with ethyl acetate. The extracts were dried ($MgSO_4$) and the solvent was evaporated. Chromatography on silica eluting with ethyl acetate/petroleum ether (60-80$^{o}$) mixtures gave the title compound (0.245g).

mp 110-1$^{o}$.

Example 61

(2-Chlorophenyl)(2,5-dimethyl-4-((dimethylamino)methyl)-1H-pyrrol-3-yl)methanone

(2-Chlorophenyl)(2,5-dimethyl-1H-pyrrol-3-yl)methanone (0.7g, 3mmoles), dimethylamine hydrochloride (0.25g,3mmoles) and paraformaldehyde (0.14g, 7mmoles) in ethanol (15ml) was heated at reflux for 18 hours. The solvent was evaporated, the residue diluted with water and this was extracted with chloroform (x2). The combined

chloroform extracts were extracted with water and the combined aqueous extracts were basified with sodium hydroxide (2N). Extraction with chloroform (x3) followed by drying ($MgSO_4$) and removal of solvent gave the title compound (0.78g). $M^+$ 290/2; NMR ($CDCl_3$) delta 2.13 (s,6H), 2.12 (s,3H), 1.67 (s,3H).

Example 62

3-(2-Chlorophenyl)-5-methyl-N-phenyl-1H-pyrazole-4-carboxamide

n-Butyl lithium (15ml of 1.6M in hexane, 24mmoles) was added to a solution at $-78^{\circ}$ of 4-bromo-3-(2-chlorophenyl)-5-methyl-1H-pyrazole (3g, 11 mmoles) in tetrahydrofuran (120ml). After 2 hours at $-5^{\circ}$, the reaction mixture was cooled to $-78^{\circ}$ and phenyl isocyanate (1.6g, 13.4mmoles) was added. After allowing the temperature to reach $0^{\circ}$, water was added. The tetrahydrofuran was evaporated and the residue extracted with chloroform. After drying ($Na_2SO_4$), the solvent was evaporated and the residue chromatographed on silica eluting with ethyl acetate/hexane mixtures. Trituration with petroleum ether (60-80$^{\circ}$) gave the title compound (2.7g), mp 101-103$^{\circ}$.

Example 63

4-(2-Chlorobenzoyl)-2,5-dimethyl-1H-pyrrole-3-carbonitrile

Chlorosulphonyl isocyanate (2.3g, 16mmoles) was added dropwise to a solution at $-78^{\circ}$ of (2-chlorophenyl) (2,5-dimethyl-1H-pyrrol-3-yl)methanone (2.5g, 10.7mmoles) in tetrahydrofuran (50ml). After 1.5 hours at $-78^{\circ}$, dimethylformamide (3ml) was added. After warming to room temperature, the reaction mixture was poured onto ice/water. The solid was filtered off and then chromatographed on silica eluting with ethyl acetate/ dichloromethane mixtures to give the title compound (0.55g).

mp 221-3°.

Example 64

i) Methyl 2-((Acetyloxy)methyl)-4-(2-chlorobenzoyl)-5-methyl-1H-pyrrole-3-carboxylate

ii) Methyl 5-((Acetyloxy)methyl)-4-(2-chlorobenzoyl)-2-methyl-1H-pyrrole-3-carboxylate

Lead tetraacetate (2.1g, 4.7mmoles) and methyl 4-(2-chlorobenzoyl)-2,5-dimethyl-1H-pyrrole-3-carboxylate (1.16g, 4mmoles) in acetic acid (5ml) were heated at 40° for 5 days. The reaction mixture was poured onto water and extracted with ethyl acetate. The organic extracted was dried ($MgSO_4$) and the solvent was evaporated. Chromatography on silica eluting with ethyl acetate/ petroleum ether (60-80°) mixtures gave the title acetates (1.06g). Separation of isomers was achieved by

HPLC on partisil eluting with methanol/dichloromethane mixtures to give the title acetates.

Isomer (i) $M^+$, 349/51; nmr ($CDCL_3$) delta 5.19 (s,2H), 3.27 (s,3H), 2.46 (s,3H) and 2.11 (s,3H).

Isomer (ii) $M^+$, 349/51; nmr ($CDCl_3$) delta 5.30 (s,2H), 3.30 (s,3H), 2.30 (s,3H) and 2.11 (s,3H).

1488K(ir)/sm/ac

We claim:

1. A compound of formula I,

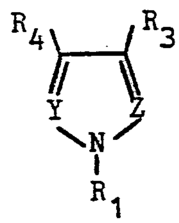

I

wherein

either one of Y and Z (which may be the same or different) represents CH, $CR_2$ or C-$COOR_{21}$, in which $R_2$ represents alkyl C 1 to C6 optionally substituted by halogen, hydroxy, amino, isothioureido or a group $R_5COO$- in which $R_5$ represents alkyl C 1 to C6, $R_2$ being optionally interrupted by O or S, and $R_{21}$ represents alkyl C 1 to C6, and the other one of Y and Z represents $CR_2$ or C-$COOR_{21}$ ,

or one of Y and Z represents N and the other CH, $CR_2$ or C-$COOR_{21}$,

$R_4$ represents $Ar_1$-B-$(W)_x$, in which

W represents C=O, S, SO, $SO_2$ or C=N-OH,

x represents 0 or 1,

B represents a single bond or $(CH_2)_p$ in which p represents 1 or 2, the group $(CH_2)_p$ being optionally substituted by alkyl C1 to C6, and

$Ar_1$ represents phenyl, naphthyl or benzofurazanyl,

 

$Ar_1$ being optionally substituted by one or more of X, $CX_3$, $OCH_nX_{3-n}$, hydroxy, alkyl C 1 to C6, Oalkyl C 1 to C6, nitro, amino, carboxy, $-SCH_2Ar_2$ or $-OCOAr_2$ in which

X represents halogen,

n represents 0, 1, 2 or 3, and

$Ar_2$ represents phenyl or phenyl substituted by hydroxy, halogen, alkyl C 1 to C6 or Oalkyl C 1 to C6,

and, in addition, when the sum of x and p is 1 or more, or when both Y and Z represent CH, $CR_2$ or $C-COOR_{21}$, then $Ar_1$ may be singly or multiply substituted by $-OCH_2Ar_2$,

$R_3$ represents $CH_2NR_6R_7$ or $COR_8$ in which $R_6$ and $R_7$, which may be the same or different, represent hydrogen, phenyl, phenyl substituted by halogen or trihalomethyl, alkyl C 1 to C6, or alkyl C 1 to C6 substituted by phenyl, and $R_8$ represents hydrogen or $NR_6R_7$,

and, in addition, when W represents C=O, S=O, $SO_2$ or C=N-OH, or when both Y and Z represent CH, $CR_2$ or $C-COOR_{21}$, then $R_8$ may also represent alkyl C 1 to C6, OH or $OR_9$ in which $R_9$ represents alkyl C 1 to C6 optionally substituted by an aryl group, and $R_3$ may represent $NO_2$, CN or halogen, and

$R_1$ represents hydrogen or alkyl C 1 to C6,

and pharmaceutically acceptable derivatives thereof.

2. A compound according to Claim 1 for use as a pharmaceutical.

3. A compound according to Claim 1 or Claim 2, wherein one of Y and Z , which may be the same or different, represents CH, $CR_2$ or $C-COOR_{21}$, and the other one of Y and Z represents $CR_2$ or $C-COOR_{21}$ .

4. A compound according to any one of the preceding claims, wherein $R_3$ represents $COOR_9$ or $NO_2$.

5. A compound according to any one of the preceding claims, wherein $R_4$ represents $Ar_1CO-$.

6. A compound according to any one of the preceding Claims, wherein $R_1$ represents hydrogen.

7. A compound according to Claim 3, wherein $R_1$ represents hydrogen, $R_3$ represents $COOR_9$ and $R_4$ represents $Ar_1CO-$ in which $Ar_1$ represents phenyl optionally singly or multiply substituted by one or more of X, $CX_3$, $-OCH_2Ar_2$ and $-OCOAr_2$ in which X represents halogen and $Ar_2$ represents phenyl optionally substituted by hydroxy.

8. A compound according to Claim 1, which is

Methyl 4-(2-Chlorobenzoyl)-2,5-dimethyl-1H-pyrrole-3-carboxylate,

Methyl 4-(2-(Trifluoromethyl)benzoyl)-2,5-dimethyl-1H-pyrrole-3-carboxylate,

Methyl 4-Benzoyl-2,5-dimethyl-1H-pyrrole-3-carboxylate,

Methyl 4-(2,5-Dichlorobenzoyl)-2,5-dimethyl-1H-pyrrole-3-carboxylate,

Methyl 4-(2,3-Dichlorobenzoyl)-2,5-dimethyl-1H-pyrrole-3-carboxylate,

Methyl 4-(3,6-Difluoro-2-(trifluoromethyl)benzoyl)-2,5-dimethyl-1H-pyrrole-3-carboxylate,

Methyl 4-(2,4-Dichlorobenzoyl)-2,5-dimethyl-1H-pyrrole-3-carboxylate,

Methyl 4-(2,6-Dichlorobenzoyl)-2,5-dimethyl-1H-pyrrole-3-carboxylate,

Methyl 4-(2-Fluorobenzoyl)-2,5-dimethyl-1H-pyrrole-3-carboxylate,

Methyl 2,5-Dimethyl-4-(pentafluorobenzoyl)-1H-pyrrole-3-carboxylate,

Methyl 4-(2-(2-Hydroxybenzoyloxy)benzoyl)-2,5-dimethyl-1H-pyrrole-3-carboxylate,

Methyl 4-(2-Iodobenzoyl)-2,5-dimethyl-1H-pyrrole-3-carboxylate,

Methyl 4-(2-Bromobenzoyl)-2,5-dimethyl-1H-pyrrole-3-carboxylate,

Methyl 4-(2-Chlorobenzoyl)-5-ethyl-2-methyl-1H-pyrrole-3-carboxylate,

Methyl 4-(2-Chlorobenzoyl)-2-methyl-5-(1-methylethyl)-

1H-pyrrole-3-carboxylate,

Methyl 4-(2-Chlorobenzoyl)-5-(1,1-dimethylethyl)-2-methyl-1H-pyrrole-3-carboxylate,

Methyl 4-(2-Chloro-5-nitrobenzoyl)-2,5-dimethyl-1H-pyrrole-3-carboxylate,

Methyl 4-(2-Chlorobenzoyl)-5-methyl-1H-pyrrole-3-carboxylate,

1-Methylethyl 4-(2-Chlorobenzoyl)-2,5-dimethyl-1H-pyrrole-3-carboxylate,

2-(2-Pyridinyl)ethyl 4-(2-Chlorobenzoyl)-2,5-dimethyl-1H-pyrrole-3-carboxylate,

Methyl 4-(2-Chlorobenzoyl)-5-methyl-1H-pyrrole-3-carboxylate,

Methyl 2-((Acetyloxy)methyl)-4-(2-chlorobenzoyl)-5-methyl-1H-pyrrole-3-carboxylate,

Methyl 5-((Acetyloxy)methyl)-4-(2-chlorobenzoyl)-2-methyl-1H-pyrrole-3-carboxylate,

or a pharmaceutically acceptable derivative of any one thereof.

9. A compound according to Claim 1, which is

Methyl 2,5-Dimethyl-4-(2-nitrobenzoyl)-1H-pyrrole-3-carboxylate,

Methyl 4-(2-Methoxybenzoyl)-2,5-dimethyl-1H-pyrrole-3-carboxylate,

Methyl 2,5-Dimethyl-4-(1-naphthalenylcarbonyl)-1H-

pyrrole-3-carboxylate,

Methyl 2,5-Dimethyl-4-(2-methylbenzoyl)-lH-pyrrole-3-carboxylate,

Methyl 4-((2-Chlorophenyl)acetyl)-2,5-dimethyl-lH-pyrrole-3-carboxylate,

Methyl 4-(4-Benzofurazanylcarbonyl)-2,5-dimethyl-lH-pyrrole-3-carboxylate,

Methyl 4-((2-Chlorophenyl)thio)-2,5-dimethyl-lH-pyrrole-3-carboxylate,

1-(4-(2-Chlorobenzoyl)-2,5-dimethyl-lH-pyrrol-3-yl)ethanone,

Methyl 2,5-Dimethyl-4-(2-((phenylmethyl)thio)benzoyl)-lH-pyrrole-3-carboxylate,

Methyl 4-(2-Carboxybenzoyl)-2,5-dimethyl-lH-pyrrole-3-carboxylate,

Ethyl 4-((2-Chlorophenyl)methyl)-2,5-dimethyl-lH-pyrrole-3-carboxylate,

2-Methyl-N-phenyl-4-(2-(trifluoromethyl)phenyl)-lH-pyrrole-3-carboxamide,

Ethyl 4-(2-Chlorophenyl)-2-methyl-lH-pyrrole-3-carboxylate,

Methyl 2-Methyl-4-(2-(trifluoromethyl)phenyl)-lH-pyrrole-3-carboxylate,

4-(2-Chlorophenyl)-2-methyl-N-phenyl-lH-pyrrole-3-carboxamide,

4-(2-Chlorophenyl)-2-methyl-3-nitro-1H-pyrrole,

Methyl 2,5-Dimethyl-4-(1-phenylethyl)-1H-pyrrole-3-carboxylate,

Methyl 2,5-Dimethyl-4-(1-naphthalenyl)-1H-pyrrole-3-carboxylate,

Methyl 3-(2-Chlorophenyl)-5-methyl-1H-pyrazole-4-carboxylate,

Methyl 3-((2-Chlorophenyl)methyl)-5-methyl-1H-pyrazole-4-carboxylate,

Methyl 4-(2-Chlorobenzoyl)-5-methyl-1H-pyrazole-3-carboxylate,

Methyl 3-(2-chlorobenzoyl)-5-methyl-1H-pyrazole-4-carboxylate,

Methyl 3-(2-Chlorobenzoyl)-1H-pyrazole-4-carboxylate,

3-Ethyl 5-methyl 4-(2-chlorobenzoyl)-1H-pyrazole-3,5-dicarboxylate,

3-Ethyl 4-methyl 5-(2-chlorobenzoyl)-1H-pyrazole-3,4-dicarboxylate,

Methyl 4-(2-Aminobenzoyl)-2,5-dimethyl-1H-pyrrole-3-carboxylate,

4-(2-Chlorobenzoyl)-2,5-dimethyl-1H-pyrrole-3-carboxylic acid,

4-(2-Chlorobenzoyl)-2,5-dimethyl-N-phenyl-1H-pyrrole-3-carboxamide,

4-(2-Chlorobenzoyl)-2,5-dimethyl-N-(phenylmethyl)-1H-

pyrrole-3-carboxamide,

4-(2-Chlorobenzoyl)-2,5-dimethyl-1H-pyrrole-3-carboxamide,

Methyl 1,2,5-trimethyl-4-(2-(trifluoromethyl)benzoyl)-1H-pyrrole-3-carboxylate,

4-(2-Chlorobenzoyl)-2,5-dimethyl-1H-pyrrole-3-carboxaldehyde,

(4-Bromo-2,5-dimethyl-1H-pyrrol-3-yl)(2-chlorophenyl)methanone,

4-Bromo-3-(2-chlorophenyl)-5-methyl-1H-pyrazole,

Methyl 4-[(2-Chlorophenyl)sulphinyl]-2,5-dimethyl-1H-pyrrole-3-carboxylate,

Methyl 4-((2-Chlorophenyl)sulphonyl)-2,5-dimethyl-1H-pyrrole-3-carboxylate,

Methyl 4-[(2-Chlorophenyl)(hydroxyimino)methyl]-2,5-dimethyl-1H-pyrrole-3-carboxylate,

(2-Chlorophenyl)(2,5-dimethyl-4-nitro-1H-pyrrol-3-yl)methanone,

3-(2-Chlorophenyl)-5-methyl-4-nitro-1H-pyrazole,

Methyl 4-(2-Hydroxybenzoyl)-2,5-dimethyl-1H-pyrrole-3-carboxylate,

Methyl 2,5-Dimethyl-4-(2-(phenylmethoxy)benzoyl)-1H-pyrrole-3-carboxylate,

(2-Chlorophenyl)(2,5-dimethyl-4-((dimethylamino)methyl)-1H-pyrrol-3-yl)methanone,

 0230110

3-(2-Chlorophenyl)-5-methyl-N-phenyl-1H-pyrazole-4-carboxamide,

4-(2-Chlorobenzoyl)-2,5-dimethyl-1H-pyrrole-3-carbonitrile,

or a pharmaceutically acceptable derivative of any one thereof.

10. A pharmaceutical composition comprising a compound according to any one of the preceding Claims in admixture with a pharmaceutically acceptable adjuvant, diluent or carrier.

11. A process for the production of a compound of formula I, according to Claim 1, or a pharmaceutically acceptable derivative thereof, which comprises

a) producing a compound of formula I in which x represents 1 and W represents S or CO by reacting a compound of formula II,

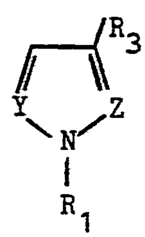

II

in which Y, Z, $R_1$ and $R_3$ are as defined above, with a compound of formula III,

$Ar_1$-B-$W_a$-$L_a$ III

in which $Ar_1$ and B are as defined above, $W_a$ represents S or CO, and $L_a$ represents a leaving group, or b) producing a compound of formula I in which $R_4$ represents $Ar_1$-B-$CH_2$ in which B represents a single bond or $-CH_2-$ optionally substituted by alkyl C 1 to C6, by reacting a compound of formula II with an aldehyde of formula IV,

$Ar_1-B_b-CHO$ IV

in which $B_b$ represents a single bond or $-CH_2-$ optionally substituted by alkyl C 1 to C6 and $Ar_1$ is as defined above, under reductive alkylation conditions, or c) producing a compound of formula I in which Z represents $CR_2$, by reacting a compound of formula V,

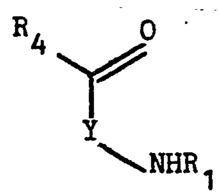

V

in which Y, $R_1$ and $R_4$ are as defined above, with a compound of formula VI,

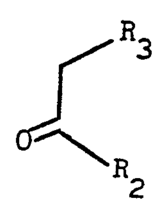

VI

in which $R_2$ and $R_3$ are as defined above, or

d) producing a compound of formula I in which Y represents N by reacting a compound of formula VII,

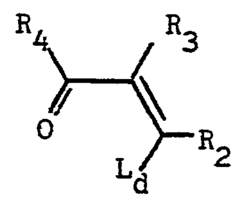

VII

in which $R_2$, $R_3$ and $R_4$ are as defined above and $L_d$ represents a leaving group, with a compound of formula VIII,

$NH_2NHR_1$ VIII

in which $R_1$ is as defined above, and where necessary separating any isomers produced, or

e) producing a compound of formula I in which $R_3$ represents CHO, halogen, $NO_2$, CN or $CH_2NR_6R_7$, in which $R_6$ and $R_7$ are as defined above, by reacting a compound of formula XI,

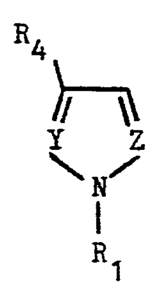

IX

in which Y, Z, $R_1$ and $R_4$ are as defined above, with an appropriate electrophilic reagent, or

f) producing a compound of formula I in which $R_3$ represents $-CONHR_6$, by reacting a compound of formula X,

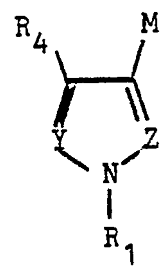

X

in which Y, Z, $R_1$ and $R_4$ are as defined above and M represents a metal, with an isocyanate of formula XI,

$$R_6-N=C=O \qquad XI$$

in which $R_6$ is as defined above, or

g) producing a compound of formula I in which $R_3$ represents $-COR_8$, in which $R_8$ represents $NR_6R_7$ or $OR_9$, in which $R_6$, $R_7$ and $R_9$ are as defined above, by reacting a compound of formula XII,

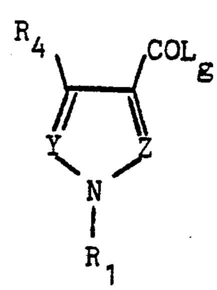

XII

in which Y, Z, $R_1$ and $R_4$ are as defined above and

$L_g$ represents a leaving group, with a compound of formula XIII,

$$R_8\text{-}H \qquad \text{XIII}$$

in which $R_8$ represents $NR_6R_7$ or $OR_9$, or

h) producing a compound of formula I in which $R_3$ represents $NO_2$ and $R_1$ represents hydrogen by rearrangement of a compound of formula XIV,

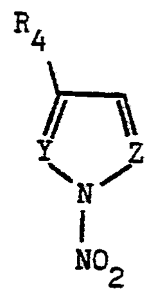

XIV

in which Y, Z and $R_4$ are as defined above, or

i) producing a compound of formula I in which $R_1$ represents hydrogen and one of Y and Z represents nitrogen and the other represents $CR_2$ or C-$COOR_{21}$ by reaction of a compound of formula XV,

$$R_4\text{-}C{\equiv}C\text{-}R_3 \qquad \text{XV}$$

in which $R_3$ and $R_4$ are as defined above with a diazo compound of formula XVI or XVII, as appropriate,

$R_2-CH=\overset{+}{N}=\overset{-}{N}$ XVI

$R_{21}OOC-CH=\overset{+}{N}=\overset{-}{N}$ XVII

in which $R_2$ and $R_{21}$ are as defined above, or

j) producing a compound of formula I in which $R_1$ represents alkyl C 1 to 6 by alkylation of the corresponding compound of formula I in which $R_1$ represents hydrogen, or

k) producing a compound of formula I in which Y represents $C-R_2$ or $C-COOR_{21}$ by reacting a compound of formula XVIII,

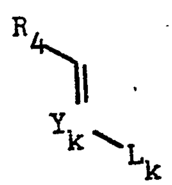

XVIII

in which $R_4$ is as defined above, $Y_k$ represents $C-R_2$ or $C-COOR_{21}$ and $L_k$ represents a leaving group, with a compound of formula XIX,

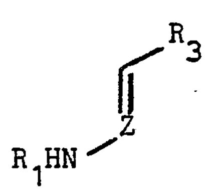

XIX

in which $R_1$, $R_3$ and Z are as defined above, or

l) producing a compound of formula I in which x represents 1 and W represents (C=N-OH) by treatment of the corresponding compound of formula I in which W represents CO with hydroxylamine, or

m) producing a compound of formula I in which x represents 1 and W represents SO or $SO_2$ by oxidation of the corresponding compound of formula I in which W represents S, or

n) producing a compound of formula I in which one of Y and Z represents CH and the other $CR_2$ by reacting a compound of formula XX,

$$R_4-CH=CH-R_3 \qquad \text{XX}$$

in which $R_3$ and $R_4$ are as defined above, with a compound of formula XXI,

$$R_2-\underset{\underset{\displaystyle L_n}{|}}{CH}-\overset{+}{N}\equiv\overset{-}{C} \qquad \text{XXI}$$

in which $R_2$ is as defined above and $L_n$ represents a leaving group, and where necessary separating any isomers produced, or

o) producing a compound of formula I in which $Ar_1$ is

substituted by $NH_2$ by reducing the corresponding compound of formula I in which $Ar_1$ is substituted by $NO_2$, or

p) producing a compound of formula I in which one of Y and Z represents CH by dealkylation of the corresponding compound of formula I in which one of Y and Z represents $C-R_2$ in which $R_2$ represents alkyl C 1 to C6, or

q) producing a compound of formula I in which $Ar_1$ is substituted by hydroxy by hydrolysis of the corresponding compound of formula I in which $Ar_1$ is substituted by Oalkyl, or

r) producing a compound of formula I in which $Ar_1$ is substituted by $-OCH_2Ar_2$ or $-OCOAr_2$ in which $Ar_2$ is as defined above by reacting the corresponding compound of formula I in which $Ar_1$ is substituted by hydroxy with a compound of formula XXII ,

$$Ar_2-X_r-L_r \qquad \text{XXII}$$

in which $Ar_2$ is as defined above, $X_r$ represents CO or $CH_2$ and $L_r$ represents a leaving group or,

s) producing a compound of formula I in which one or both of Y and Z represent $C-R_2$ in which $R_2$ represents alkyl C 1 to C6 substituted by a group $R_5COO-$ by alkanoyloxylation of the corresponding compound of formula

I in which $R_2$ represents alkyl C 1 to C6, or

t) producing a compound of formula I in which $R_3$ represents COOH by hydrolysis of the corresponding compound of formula I in which $R_3$ represents $COOR_9$, or

u) producing a compound of formula I in which at least one of Y and Z represents $C-R_2$ in which $R_2$ represents alkyl C 1 to C6 substituted by hydroxy by reacting the corresponding compound of formula I in which $R_2$ represents alkyl C 1 to C6 substituted by $R_5COO-$ with a hydroxylic base, and optionally producing a corresponding compound in which $R_2$ is substituted by Oalkyl C 1 to C6 by conducting the reaction in the presence of the corresponding alkanol,

and, where necessary or desired, converting the compound of formula I into a pharmaceutically acceptable derivative, or vice versa.

1543K/saj

Claims for Contracting States AT, ES and GR

We claim:

1. A process for the production of a compound of formula I,

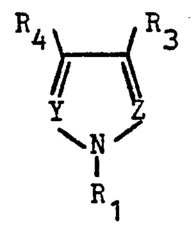

I

wherein

either one of Y and Z (which may be the same or different) represents CH, $CR_2$ or $C-COOR_{21}$, in which $R_2$ represents alkyl C 1 to C6 optionally substituted by halogen, hydroxy, amino, isothioureido or a group $R_5COO-$ in which $R_5$ represents alkyl C 1 to C6, $R_2$ being optionally interrupted by O or S, and $R_{21}$ represents alkyl C 1 to C6, and the other one of Y and Z represents $CR_2$ or $C-COOR_{21}$ ,

or one of Y and Z represents N and the other CH, $CR_2$ or $C-COOR_{21}$,

$R_4$ represents $Ar_1-B-(W)_x$, in which

W represents C=O, S, SO, $SO_2$ or C=N-OH,

x represents 0 or 1,

B represents a single bond or $(CH_2)_p$ in which p represents 1 or 2, the group $(CH_2)_p$ being optionally

substituted by alkyl C1 to C6, and

$Ar_1$ represents phenyl, naphthyl or benzofurazanyl, $Ar_1$ being optionally substituted by one or more of X, $CX_3$, $OCH_nX_{3-n}$, hydroxy, alkyl C 1 to C6, Oalkyl C 1 to C6, nitro, amino, carboxy, $-SCH_2Ar_2$ or $-OCOAr_2$ in which

X represents halogen,

n represents 0, 1, 2 or 3, and

$Ar_2$ represents phenyl or phenyl substituted by hydroxy, halogen, alkyl C 1 to C6 or Oalkyl C 1 to C6,

and, in addition, when the sum of x and p is 1 or more, or when both Y and Z represent CH, $CR_2$ or $C-COOR_{21}$, then $Ar_1$ may be singly or multiply substituted by $-OCH_2Ar_2$,

$R_3$ represents $CH_2NR_6R_7$ or $COR_8$ in which $R_6$ and $R_7$, which may be the same or different, represent hydrogen, phenyl, phenyl substituted by halogen or trihalomethyl, alkyl C 1 to C6, or alkyl C 1 to C6 substituted by phenyl, and $R_8$ represents hydrogen or $NR_6R_7$,

and, in addition, when W represents C=O, S=O, $SO_2$ or C=N-OH, or when both Y and Z represent CH, $CR_2$ or $C-COOR_{21}$, then $R_8$ may also represent alkyl C 1 to C6, OH or $OR_9$ in which $R_9$ represents alkyl C 1 to C6 optionally substituted by an aryl group, and $R_3$ may

represent $NO_2$, CN or halogen, and

$R_1$ represents hydrogen or alkyl C 1 to C6,

and pharmaceutically acceptable derivatives thereof, which comprises

a) producing a compound of formula I in which x represents 1 and W represents S or CO by reacting a compound of formula II,

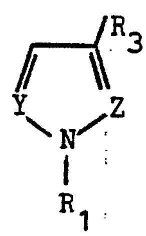

II

in which Y, Z, $R_1$ and $R_3$ are as defined above, with a compound of formula III,

$$Ar_1\text{-}B\text{-}W_a\text{-}L_a \qquad III$$

in which $Ar_1$ and B are as defined above, $W_a$ represents S or CO, and $L_a$ represents a leaving group, or

b) producing a compound of formula I in which $R_4$ represents $Ar_1\text{-}B\text{-}CH_2$ in which B represents a single bond or $-CH_2-$ optionally substituted by alkyl C 1 to C6, by reacting a compound of formula II with an aldehyde of formula IV,

$Ar_1-B_b-CHO$ IV

in which $B_b$ represents a single bond or $-CH_2-$ optionally substituted by alkyl C 1 to C6 and $Ar_1$ is as defined above, under reductive alkylation conditions, or

c) producing a compound of formula I in which Z represents $CR_2$, by reacting a compound of formula V,

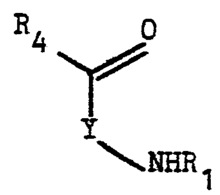

V

in which Y, $R_1$ and $R_4$ are as defined above, with a compound of formula VI,

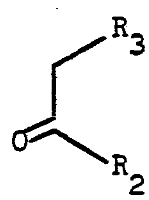

VI

in which $R_2$ and $R_3$ are as defined above, or

d) producing a compound of formula I in which Y represents N by reacting a compound of formula VII,

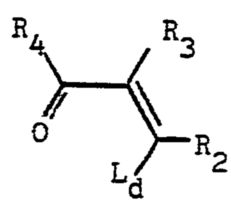

VII

in which $R_2$, $R_3$ and $R_4$ are as defined above and $L_d$ represents a leaving group, with a compound of formula VIII,

$$NH_2NHR_1 \qquad VIII$$

in which $R_1$ is as defined above, and where necessary separating any isomers produced, or

e) producing a compound of formula I in which $R_3$ represents CHO, halogen, $NO_2$, CN or $CH_2NR_6R_7$, in which $R_6$ and $R_7$ are as defined above, by reacting a compound of formula XI,

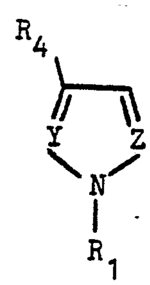

IX

in which Y, Z, $R_1$ and $R_4$ are as defined above, with an appropriate electrophilic reagent, or

f) producing a compound of formula I in which $R_3$ represents $-CONHR_6$, by reacting a compound of formula X,

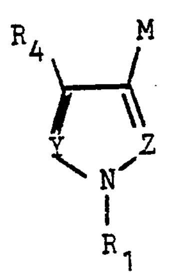

X

in which Y, Z, $R_1$ and $R_4$ are as defined above and M represents a metal, with an isocyanate of formula XI,

$R_6$-N=C=O XI

in which $R_6$ is as defined above, or

g) producing a compound of formula I in which $R_3$ represents $-COR_8$, in which $R_8$ represents $NR_6R_7$ or $OR_9$, in which $R_6$, $R_7$ and $R_9$ are as defined above, by reacting a compound of formula XII,

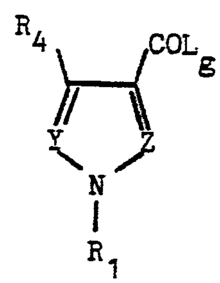

XII

in which Y, Z, $R_1$ and $R_4$ are as defined above and $L_g$ represents a leaving group, with a compound of formula XIII,

$R_8$-H XIII

in which $R_8$ represents $NR_6R_7$ or $OR_9$, or

h) producing a compound of formula I in which $R_3$ represents $NO_2$ and $R_1$ represents hydrogen by rearrangement of a compound of formula XIV,

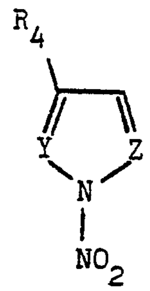

XIV

in which Y, Z and $R_4$ are as defined above, or

i) producing a compound of formula I in which $R_1$ represents hydrogen and one of Y and Z represents nitrogen and the other represents $CR_2$ or $C-COOR_{21}$ by reaction of a compound of formula XV,

$$R_4-C\equiv C-R_3 \qquad XV$$

in which $R_3$ and $R_4$ are as defined above with a diazo compound of formula XVI or XVII, as appropriate,

$$R_2-CH=\overset{+}{N}=\overset{-}{N} \qquad XVI$$

$$R_{21}OOC-CH=\overset{+}{N}=\overset{-}{N} \qquad XVII$$

in which $R_2$ and $R_{21}$ are as defined above, or

j) producing a compound of formula I in which $R_1$ represents alkyl C 1 to 6 by alkylation of the corresponding compound of formula I in which $R_1$ represents hydrogen, or

k) producing a compound of formula I in which Y represents $C-R_2$ or $C-COOR_{21}$ by reacting a compound of formula XVIII,

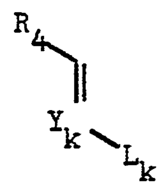

XVIII

in which $R_4$ is as defined above, $Y_k$ represents $C-R_2$ or $C-COOR_{21}$ and $L_k$ represents a leaving group, with a compound of formula XIX,

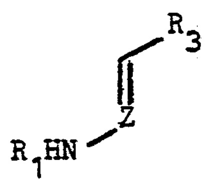

XIX

in which $R_1$, $R_3$ and Z are as defined above, or

l) producing a compound of formula I in which x represents 1 and W represents (C=N-OH) by treatment of the corresponding compound of formula I in which W represents CO with hydroxylamine, or

m) producing a compound of formula I in which x represents 1 and W represents SO or $SO_2$ by oxidation of the corresponding compound of formula I in which W represents S, or

n) producing a compound of formula I in which one of Y and Z represents CH and the other $CR_2$ by reacting a compound of formula XX,

$$R_4\text{-}CH\text{=}CH\text{-}R_3 \qquad \text{XX}$$

in which $R_3$ and $R_4$ are as defined above, with a compound of formula XXI,

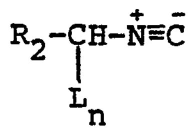

XXI

in which $R_2$ is as defined above and $L_n$ represents a leaving group, and where necessary separating any isomers produced, or

o) producing a compound of formula I in which $Ar_1$ is substituted by $NH_2$ by reducing the corresponding compound of formula I in which $Ar_1$ is substituted by $NO_2$, or

p) producing a compound of formula I in which one of Y and Z represents CH by dealkylation of the corresponding compound of formula I in which one of Y and Z represents C-$R_2$ in which $R_2$ represents alkyl C 1 to C6, or

q) producing a compound of formula I in which $Ar_1$ is substituted by hydroxy by hydrolysis of the corresponding

compound of formula I in which $Ar_1$ is substituted by Oalkyl, or

r) producing a compound of formula I in which $Ar_1$ is substituted by $-OCH_2Ar_2$ or $-OCOAr_2$ in which $Ar_2$ is as defined above by reacting the corresponding compound of formula I in which $Ar_1$ is substituted by hydroxy with a compound of formula XXII ,

$$Ar_2-X_r-L_r \qquad XXII$$

in which $Ar_2$ is as defined above, $X_r$ represents CO or $CH_2$ and $L_r$ represents a leaving group or,

s) producing a compound of formula I in which one or both of Y and Z represent $C-R_2$ in which $R_2$ represents alkyl C 1 to C6 substituted by a group $R_5COO-$ by alkanoyloxylation of the corresponding compound of formula I in which $R_2$ represents alkyl C 1 to C6, or

t) producing a compound of formula I in which $R_3$ represents COOH by hydrolysis of the corresponding compound of formula I in which $R_3$ represents $COOR_9$, or

u) producing a compound of formula I in which at least one of Y and Z represents $C-R_2$ in which $R_2$ represents alkyl C 1 to C6 substituted by hydroxy by reacting the corresponding compound of formula I in which $R_2$ represents alkyl C 1 to C6 substituted by $R_5COO-$ with a

hydroxylic base, and optionally producing a corresponding compound in which $R_2$ is substituted by Oalkyl C 1 to C6 by conducting the reaction in the presence of the corresponding alkanol,

and, where necessary or desired, converting the compound of formula I into a pharmaceutically acceptable derivative, or vice versa.

2. A process according to Claim 1, wherein one of Y and Z , which may be the same or different, represents CH, $CR_2$ or $C-COOR_{21}$, and the other one of Y and Z represents $CR_2$ or $C-COOR_{21}$ .

3. A process according to any one of the preceding claims, wherein $R_3$ represents $COOR_9$ or $NO_2$.

4. A process according to any one of the preceding claims, wherein $R_4$ represents $Ar_1CO-$.

5. A process according to any one of the preceding Claims, wherein $R_1$ represents hydrogen.

6. A process according to Claim 2, wherein $R_1$ represents hydrogen, $R_3$ represents $COOR_9$ and $R_4$ represents $Ar_1CO-$ in which $Ar_1$ represents phenyl optionally singly or multiply substituted by one or more of X, $CX_3$, $-OCH_2Ar_2$ and $-OCOAr_2$ in which X represents halogen and $Ar_2$ represents phenyl optionally substituted by hydroxy.

7. A process according to Claim 1, wherein the compound

of formula I is

Methyl 4-(2-Chlorobenzoyl)-2,5-dimethyl-1H-pyrrole-3-carboxylate,

Methyl 4-(2-(Trifluoromethyl)benzoyl)-2,5-dimethyl-1H-pyrrole-3-carboxylate,

Methyl 4-Benzoyl-2,5-dimethyl-1H-pyrrole-3-carboxylate,

Methyl 4-(2,5-Dichlorobenzoyl)-2,5-dimethyl-1H-pyrrole-3-carboxylate,

Methyl 4-(2,3-Dichlorobenzoyl)-2,5-dimethyl-1H-pyrrole-3-carboxylate,

Methyl 4-(3,6-Difluoro-2-(trifluoromethyl)benzoyl)-2,5-dimethyl-1H-pyrrole-3-carboxylate,

Methyl 4-(2,4-Dichlorobenzoyl)-2,5-dimethyl-1H-pyrrole-3-carboxylate,

Methyl 4-(2,6-Dichlorobenzoyl)-2,5-dimethyl-1H-pyrrole-3-carboxylate,

Methyl 4-(2-Fluorobenzoyl)-2,5-dimethyl-1H-pyrrole-3-carboxylate,

Methyl 2,5-Dimethyl-4-(pentafluorobenzoyl)-1H-pyrrole-3-carboxylate,

Methyl 4-(2-(2-Hydroxybenzoyloxy)benzoyl)-2,5-dimethyl-1H-pyrrole-3-carboxylate,

Methyl 4-(2-Iodobenzoyl)-2,5-dimethyl-1H-pyrrole-3-carboxylate,

Methyl 4-(2-Bromobenzoyl)-2,5-dimethyl-1H-pyrrole-3-carboxylate,

Methyl 4-(2-Chlorobenzoyl)-5-ethyl-2-methyl-1H-pyrrole-3-carboxylate,

Methyl 4-(2-Chlorobenzoyl)-2-methyl-5-(1-methylethyl)-1H-pyrrole-3-carboxylate,

Methyl 4-(2-Chlorobenzoyl)-5-(1,1-dimethylethyl)-2-methyl-1H-pyrrole-3-carboxylate,

Methyl 4-(2-Chloro-5-nitrobenzoyl)-2,5-dimethyl-1H-pyrrole-3-carboxylate,

Methyl 4-(2-Chlorobenzoyl)-5-methyl-1H-pyrrole-3-carboxylate,

1-Methylethyl 4-(2-Chlorobenzoyl)-2,5-dimethyl-1H-pyrrole-3-carboxylate,

2-(2-Pyridinyl)ethyl 4-(2-Chlorobenzoyl)-2,5-dimethyl-1H-pyrrole-3-carboxylate,

Methyl 4-(2-Chlorobenzoyl)-5-methyl-1H-pyrrole-3-carboxylate,

Methyl 2-((Acetyloxy)methyl)-4-(2-chlorobenzoyl)-5-methyl-1H-pyrrole-3-carboxylate,

Methyl 5-((Acetyloxy)methyl)-4-(2-chlorobenzoyl)-2-methyl-1H-pyrrole-3-carboxylate,

or a pharmaceutically acceptable derivative of any one thereof.

8. A process according to Claim 1, wherein the compound

of formula I is

Methyl 2,5-Dimethyl-4-(2-nitrobenzoyl)-1H-pyrrole-3-carboxylate,

Methyl 4-(2-Methoxybenzoyl)-2,5-dimethyl-1H-pyrrole-3-carboxylate,

Methyl 2,5-Dimethyl-4-(1-naphthalenylcarbonyl)-1H-pyrrole-3-carboxylate,

Methyl 2,5-Dimethyl-4-(2-methylbenzoyl)-1H-pyrrole-3-carboxylate,

Methyl 4-((2-Chlorophenyl)acetyl)-2,5-dimethyl-1H-pyrrole-3-carboxylate,

Methyl 4-(4-Benzofurazanylcarbonyl)-2,5-dimethyl-1H-pyrrole-3-carboxylate,

Methyl 4-((2-Chlorophenyl)thio)-2,5-dimethyl-1H-pyrrole-3-carboxylate,

1-(4-(2-Chlorobenzoyl)-2,5-dimethyl-1H-pyrrol-3-yl)ethanone,

Methyl 2,5-Dimethyl-4-(2-((phenylmethyl)thio)benzoyl)-1H-pyrrole-3-carboxylate,

Methyl 4-(2-Carboxybenzoyl)-2,5-dimethyl-1H-pyrrole-3-carboxylate,

Ethyl 4-((2-Chlorophenyl)methyl)-2,5-dimethyl-1H-pyrrole-3-carboxylate,

2-Methyl-N-phenyl-4-(2-(trifluoromethyl)phenyl)-1H-pyrrole-3-carboxamide,

Ethyl 4-(2-Chlorophenyl)-2-methyl-1H-pyrrole-3-carboxylate,

Methyl 2-Methyl-4-(2-(trifluoromethyl)phenyl)-1H-pyrrole-3-carboxylate,

4-(2-Chlorophenyl)-2-methyl-N-phenyl-1H-pyrrole-3-carboxamide,

4-(2-Chlorophenyl)-2-methyl-3-nitro-1H-pyrrole,

Methyl 2,5-Dimethyl-4-(1-phenylethyl)-1H-pyrrole-3-carboxylate,

Methyl 2,5-Dimethyl-4-(1-naphthalenyl)-1H-pyrrole-3-carboxylate,

Methyl 3-(2-Chlorophenyl)-5-methyl-1H-pyrazole-4-carboxylate,

Methyl 3-((2-Chlorophenyl)methyl)-5-methyl-1H-pyrazole-4-carboxylate,

Methyl 4-(2-Chlorobenzoyl)-5-methyl-1H-pyrazole-3-carboxylate,

Methyl 3-(2-chlorobenzoyl)-5-methyl-1H-pyrazole-4-carboxylate,

Methyl 3-(2-Chlorobenzoyl)-1H-pyrazole-4-carboxylate,

3-Ethyl 5-methyl 4-(2-chlorobenzoyl)-1H-pyrazole-3,5-dicarboxylate,

3-Ethyl 4-methyl 5-(2-chlorobenzoyl)-1H-pyrazole-3,4-dicarboxylate,

Methyl 4-(2-Aminobenzoyl)-2,5-dimethyl-1H-pyrrole-3-

carboxylate,

4-(2-Chlorobenzoyl)-2,5-dimethyl-1H-pyrrole-3-carboxylic acid,

4-(2-Chlorobenzoyl)-2,5-dimethyl-N-phenyl-1H-pyrrole-3-carboxamide,

4-(2-Chlorobenzoyl)-2,5-dimethyl-N-(phenylmethyl)-1H-pyrrole-3-carboxamide,

4-(2-Chlorobenzoyl)-2,5-dimethyl-1H-pyrrole-3-carboxamide,

Methyl 1,2,5-trimethyl-4-(2-(trifluoromethyl) benzoyl)-1H-pyrrole-3-carboxylate,

4-(2-Chlorobenzoyl)-2,5-dimethyl-1H-pyrrole-3-carboxaldehyde,

(4-Bromo-2,5-dimethyl-1H-pyrrol-3-yl)(2-chlorophenyl) methanone,

4-Bromo-3-(2-chlorophenyl)-5-methyl-1H-pyrazole,

Methyl 4-[(2-Chlorophenyl)sulphinyl]-2,5-dimethyl-1H-pyrrole-3-carboxylate,

Methyl 4-((2-Chlorophenyl)sulphonyl)-2,5-dimethyl-1H-pyrrole-3-carboxylate,

Methyl 4-[(2-Chlorophenyl)(hydroxyimino)methyl]-2,5-dimethyl-1H-pyrrole-3-carboxylate,

(2-Chlorophenyl)(2,5-dimethyl-4-nitro-1H-pyrrol-3-yl) methanone,

3-(2-Chlorophenyl)-5-methyl-4-nitro-1H-pyrazole,

Methyl 4-(2-Hydroxybenzoyl)-2,5-dimethyl-1H-pyrrole-3-carboxylate,

Methyl 2,5-Dimethyl-4-(2-(phenylmethoxy)benzoyl)-1H-pyrrole-3-carboxylate,

(2-Chlorophenyl)(2,5-dimethyl-4-((dimethylamino)methyl)-1H-pyrrol-3-yl)methanone,

3-(2-Chlorophenyl)-5-methyl-N-phenyl-1H-pyrazole-4-carboxamide,

4-(2-Chlorobenzoyl)-2,5-dimethyl-1H-pyrrole-3-carbonitrile,

or a pharmaceutically acceptable derivative of any one thereof.

1790K/saj

0230110

Application number

European Patent Office

# EUROPEAN SEARCH REPORT

EP 86309235.9

| Category | Citation of document with indication, where appropriate, of relevant passages | Relevant to claim | CLASSIFICATION OF THE APPLICATION (Int. Cl 4) |
|---|---|---|---|
| A | AT - B - 374 176 (DAINIPPON PHARMACEUTICAL CO., LTD.)<br>* Page 2 *<br>-- | 1,10, 11 | C 07 D 207/337<br>C 07 D 231/14<br>C 07 D 401/06<br>C 07 D 413/06<br>A 61 K 31/395 |
| A | AT - B - 374 794 (DAINIPPON PHARMACEUTICAL CO., LTD.)<br>* Page 2 *<br>-- | 1,10, 11 | |
| A | GB - A - 2 122 188 (NISSAN CHEMICAL INDUSTRIES LTD.)<br>* Abstract *<br>---- | 1 | TECHNICAL FIELDS SEARCHED (Int. Cl 4)<br>C 07 D 207/00<br>C 07 D 231/00<br>C 07 D 401/00<br>C 07 D 413/00 |

The present search report has been drawn up for all claims

| Place of search | Date of completion of the search | Examiner |
|---|---|---|
| VIENNA | 02-03-1987 | HEIN |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82